(19) Europäisches Patentamt
European Patent Office
Office européen des brevets

(11) Publication number: 0 158 349 B1

(12) **EUROPEAN PATENT SPECIFICATION**

(45) Date of publication of patent specification: **14.03.90**

(21) Application number: **85104388.5**

(22) Date of filing: **11.04.85**

(51) Int. Cl.[5]: **C 01 B 25/45,** C 01 B 35/14, B 01 J 29/04, C 10 G 11/04, C 10 G 49/02, C 07 C 5/02, C 07 C 2/54, C 10 G 35/06, C 07 C 5/22, C 07 C 5/41

# (54) Molecular sieve compositions.

(30) Priority: **13.04.84 US 599978**

(43) Date of publication of application: **16.10.85 Bulletin 85/42**

(45) Publication of the grant of the patent: **14.03.90 Bulletin 90/11**

(84) Designated Contracting States: **BE DE FR GB IT NL**

(56) References cited: **EP-A-0 043 562**

(73) Proprietor: **UOP**
**25 East Algonquin Road**
**Des Plaines Illinois 60017-5017 (US)**

(72) Inventor: **Lok, Brent Mai Tak**
**17A Deerwood Drive**
**New City New York 10956 (US)**
Inventor: **Marcus, Bonita Kristoffersen**
**49 Meadow Place**
**Rye New York 10580 (US)**
Inventor: **Messina, Celeste Anne**
**20 Somerstown Road**
**Ossining New York 10562 (US)**
Inventor: **Patton, Robert Lyle**
**87 Harris Road**
**Katonah New York 10536 (US)**
Inventor: **Wilson, Stephen Thomas**
**1024 E. Main Street**
**Shrub Oak New York 10588 (US)**
Inventor: **Flanigen, Edith Marie**
**502 Woodland Hills Road**
**White Plains New York 10603 (US)**

(74) Representative: **Eggert, Hans-Gunther, Dr.**
**Räderscheidtstrasse 1**
**D-5000 Köln 41 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 158 349 B1

**Description**

Field of the invention

The instant invention relates to a novel class of crystalline microporous molecular sieves, to the method of their preparation and to their use as adsorbents and catalysts. The invention relates to novel molecular sieves having at least four elements capable of forming framework tetrahedral oxide units. These compositions may be prepared hydrothermally from gels containing reactive compounds of aluminum and phosphorus and at least one element from each of two groups of elements, as hereinafter set forth, capable of forming a framework tetrahedral oxide, and preferably at least one organic templating agent which functions in part to determine the course of the crystallization mechanism and the structure of the crystalline product.

Background of the invention

Molecular sieves of the crystalline aluminosilicate zeolite type are well known in the art and now comprise over 150 species of both naturally occurring and synthetic compositions. In general the crystalline zeolites are formed from corner-sharing $AlO_2$ and $SiO_2$ tetrahedra and are characterized by having pore openings of uniform dimensions, having a significant ion-exchange capacity and being capable of reversibly desorbing an adsorbed phase which is dispersed throughout the internal voids of the crystal without displacing any atoms which make up the permanent crystal structure.

Other crystalline microporous compositions which are not zeolitic, i.e. do not contain only $AlO_2$ and $SiO_2$ tetrahedra as essential framework constituents, but which exhibit the ion-exchange and/or adsorption characteristics of the zeolites are also known. Metal organosilicates which are said to possess ion-exchange properties, have uniform pores and are capable of reversibly adsorbing molecules having molecular diameters of about 6Å or less, are reported in U.S. Patent No. 3,941,871 issued March 2, 1976 to Dwyer et al. A pure silica polymorph, silicalite, having molecular sieving properties and a neutral framework containing neither cations nor cation sites is disclosed in U.S. Patent No. 4,061,724 issued December 6, 1977 to R. W. Grose et al.

A recently reported class of microporous compositions and the first framework oxide molecular sieves synthesized without silica, are the crystalline aluminophosphate compositions disclosed in U.S. Patent No. 4,310,440 issued January 12, 1982 to Wilson et al. These materials are formed from $AlO_2$ and $PO_2$ tetrahedra and have electrovalently neutral frameworks as in the case of silica polymorphs. Unlike the silica molecular sieve, silicalite, which is hydrophobic due to the absence of extra-structural cations, the aluminophosphate molecular sieves are moderately hydrophilic, apparently due to the difference in electronegativity between aluminum and phosphorus. Their intracrystalline pore volumes and pore diameters are comparable to those known for zeolites and silica molecular sieves.

In copending EP—A—0 103 117, there is described a novel class of silicon-substituted aluminophosphates which are both microporous and crystalline. The materials have a three dimensional crystal framework of $PO_2^+$, $AlO_2^-$ and $SiO_2$ tetrahedral units and, exclusive of any alkali metal or calcium which may optionally be present, an as-synthesized empirical chemical composition on an anhydrous basis of:

$$mR:(Si_xAl_yP_z)O_2$$

wherein "R" represents at least one organic templating agent present in the intracrystalline pore system: "m" represents the moles of "R" present per mole of $(Si_xAl_yP_z)O_2$ and has a value of from zero to 0.3, the maximum value in each case depending upon the molecular dimensions of the templating agent and the available void volume of the pore system of the particular silicoaluminophosphate species involved; and "x", "y", and "z" represent the mole fractions of silicon, aluminum and phosphorus, respectively, present as tetrahedral oxides. The minimum value for each of "x", "y", and "z" is 0.01 and preferably 0.02. The maximum value for "x" is 0.98; for "y" is 0.60; and for "z" is 0.52. These silicoaluminophosphates exhibit several physical and chemical properties which are characteristic of aluminosilicate zeolites and aluminophosphates.

In copending EP—A—0 121 232, there is described a novel class of titanium-containing molecular sieves whose chemical composition in the as-synthesized and anhydrous form is represented by the unit empirical formula:

$$mR:(Ti_xAl_yP_z)O_2$$

wherein "R" represents at least one organic templating agent present in the intracrystalline pore system; "m" represents the moles of "R" present per mole of $(Ti_xAl_yP_z)O_2$ and has a value of between zero and about 5.0; and "x", "y" and "z" represent the mole fractions of titanium, aluminum and phosphorus, respectively, present as tetrahedral oxides.

In copending EP—A—0 132 708, there is described a novel class of crystalline metal aluminophosphates having three-dimensional microporous framework structures of $MO_2$, $AlO_2$, and $PO_2$ tetrahedral units and having an empirical chemical composition on an anhydrous basis expressed by the formula:

$$mR:(M_xAl_yP_z)O_2$$

wherein "R" represents at least one organic templating agent present in the intracrystalline pore system; "m" represents the moles of "R" present per mole of $(M_xAl_yP_z)O_2$ and has a value of from zero to 0.3; "M" represents at least one metal of the group magnesium, manganese, zinc and cobalt; "x", "y" and "z" represent the mole fraction of the metal "M", aluminum and phosphorus, respectively, present as tetrahedral oxides.

In copending EP—A—0 131 946, there is described a novel class of crystalline ferroaluminophosphates having a three-dimensional microporous framework structure of $FeO_2$, $AlO_2$ and $PO_2$ tetrahedral units and having an empirical chemical composition on an anhydrous basis expressed by the formula

$$mR:(Fe_xAl_yP_z)O_2$$

wherein "R" represents at least one organic templating agent present in the intracrystalline pore system; "m" represents the moles of "R" present per mole of $(Fe_xAl_yP_z)O_2$ and has a value of from zero to 0.3; and "x", "y" and "z" represent the mole fraction of the iron, aluminum and phosphorus, respectively, present as tetrahedral oxides.

The instant invention relates to new molecular sieve compositions comprising at least two elements in the form of "$MO_2^n$" tetrahedral units, as hereinafter discussed, capable of forming framework tetrahedral oxide units with $AlO_2^-$ and $PO_2^+$ tetrahedral oxide units.

Description of the figures

Fig. 1 is a ternary diagram wherein parameters relating to the instant compositions are set forth as mole fractions.

Fig. 2 is a ternary diagram wherein parameters relating to preferred compositions are set forth as mole fractions.

Fig. 3 is a ternary diagram wherein parameters relating to the reaction mixtures employed in the preparation of the compositions of this invention are set forth as mole fractions.

Summary of the invention

The instant invention relates to a new class of crystalline molecular sieves in which at least two elements capable of forming three-dimensional microporous framework form crystal framework structures of $AlO_2^-$, $PO_2^+$ and $MO_2^n$ tetrahedral oxide units wherein "$MO_2^n$" represents at least two different elements present as tetrahedral units "$MO_2^n$" with charge "n" where "n" may be −3, −2, −1, 0 or +1. These new molecular sieves exhibit ion-exchange, adsorption and catalytic properties and accordingly find wide use as adsorbents and catalysts. The members of this novel class of compositions have crystal framework structures of $AlO_2^-$, $PO_2^+$ and $MO_2^n$ tetrahedral units and have an empirical chemical composition on an anhydrous basis expressed by the formula:

$$mR:(M_xAl_yP_z)O_2$$

wherein "R" represents at least one organic templating agent present in the intracrystalline pore system; "m" represents the molar amount of "R" present per mole of $(M_xAl_yP_z)O_2$; "M" represents at least two elements capable of forming framework tetrahedral oxides; and "x", "y" and "z" represent the mole fractions of "M", aluminum and phosphorus, respectively, present as tetrahedral oxides. "M" is at least two different elements ($M_1$ and $M_2$) such that the molecular sieves contain at least two framework tetrahedral units in addition to $AlO_2^-$ and $PO_2^+$. "M" is at least one element selected from the group consisting of arsenic, beryllium, boron, chromium, gallium, germanium, lithium and vanadium and is at least one element selected from the group consisting of cobalt, iron, magnesium, manganese, titanium and zinc.

The molecular sieves of the instant invention will be generally referred to herein by the acronym or "ELAPO" to designate elements "M" in a framework of $AlO_2^-$, $PO_2^+$ and $MO_2^n$ tetrahedral units. Actual class members will be identified by replacing the "EL" of the acronym with the elements present as $MO_2^n$ tetrahedral units. For example, "MgBeAPO" designates a molecular sieve comprised of $AlO_2^-$, $PO_2^+$, $MgO_2^{-2}$ and $BeO_2^{-2}$ tetrahedral units. To identify various structural species which make up each of the subgeneric classes, each species is assigned a number and is identified as "ELAPO-i" wherein "i" is an integer. The given species designation is not intended to denote a similarity in structure to any other species denominated by a similar identification system.

Detailed description of the invention

The instant invention relates to a new class of molecular sieves in which at least two elements capable of forming framework tetrahedral oxides are provided to form crystal framework structures of $AlO_2^-$, $PO_2^+$ and $MO_2^n$ tetrahedral wherein "M" represents at least two elements capable of forming tetrahedral units "$M_1O_2^n$" and "$M_2O_2^n$" where "$M_1$" and "$M_2$" are two different elements, where "n" is −3, −2, −1, 0 or +1 and where "$M_1$" is at least one element selected from the group consisting of arsenic, beryllium, boron,

chromium, gallium, germanium, lithium and vanadium and "$M_2$" is at least one element selected from the group consisting of cobalt, iron, magnesium, manganese, titanium and zinc. These new molecular sieves exhibit ion-exchange, adsorption and catalytic properties and accordingly find wide use as adsorbents and catalysts.

The members of this novel class of compositions have crystalline three-dimensional microporous framework structures of $AlO_2^-$, $PO_2^+$ and $MO_2^n$ tetrahedral units and have an empirical chemical composition on an anhydrous basis expressed by the formula:

$$mR:(M_xAl_yP_z)O_2;$$

wherein "R" represents at least one organic templating agent present in the intracrystalline pore system; "m" represents the molar amount of "R" present per mole of $(M_xAl_yP_z)O_2$ and has a value of zero to about 0.3; "M" represents at least two elements capable of forming framework tetrahedral oxides where "M" is at least one element selected from the group consisting of arsenic, beryllium, boron, chromium, gallium, germanium, lithium and vanadium; and at least one element selected from the group consisting of cobalt, iron, magnesium, manganese, titanium, and zinc.

The relative amounts of elements "M", aluminum and phosphorus are expressed by the empirical chemical formula (anhydrous):

$$mR:(M_xAl_yP_z)O_2$$

where "x", "y" and "z" represent the mole fractions of said "M", aluminum and phosphorus. The individual mole fractions of each "M" ($M_1$, $M_2$, $M_3$, etc.) may be represented by "$x_1$", "$x_2$", "$x_3$", etc. wherein "$x_1$", "$x_2$", and "$x_3$", and etc. represent the individual mole fractions of elements $M_1$, $M_2$, $M_3$, and etc. for "M" as above defined. The values of "$x_1$", "$x_2$", "$x_3$", etc. are as defined for "x", hereinafter, where "$x_1$"+"$x_2$"+"$x_3$" . . . ="x" and where $x_1$, $x_2$, $x_3$, etc. are each at least 0.01.

The molecular sieves of the instant invention have crystalline three-dimensional microporous framework structures of $MO_2^n$, $AlO_2^-$ and $PO_2^+$ tetrahedral units having an empirical chemical composition on an anhydrous basis expressed by the formula:

$$mR:(M_xAl_yP_z)O_2$$

wherein "R" represents at least one organic templating agent present in the intracrystalline pore system; "m" represents a molar amount of "R" present per mole of $(M_xAl_yP_z)O_2$ and has a value of zero to about 0.3; "M" represents at least two different elements capable of forming framework tetrahedral oxides, as hereinbefore defined, and "x", "y" and "z" represent the mole fractions of "M", aluminum and phosphorus, respectively, present as tetrahedral oxides; said mole fractions "x", "y" and "z" being generally defined as within the pentagonal compositional area defined by points A, B, C, D and E of the ternary diagram which is Fig. 1 of the drawings. The points A, B, C, D and E represent the following values for "x", "y", and "z":

| | Mole fraction | | |
|---|---|---|---|
| Point | x | y | z |
| A | 0.02 | 0.60 | 0.38 |
| B | 0.02 | 0.38 | 0.60 |
| C | 0.39 | 0.01 | 0.60 |
| D | 0.98 | 0.01 | 0.01 |
| E | 0.39 | 0.60 | 0.01 |

In a preferred sub-class of the ELAPOs of this invention, the values of "x", "y" and "z" in the formula above are within the hexagonal compositional area defined by the points a, b, c, d, e and f of the ternary diagram which is Fig. 2 of the drawings, the said points a, b, c, d, e and f representing the following values for "x", "y" and "z":

| | Mole fraction | | |
|---|---|---|---|
| Point | x | y | z |
| a | 0.02 | 0.60 | 0.38 |
| b | 0.02 | 0.38 | 0.60 |
| c | 0.39 | 0.01 | 0.60 |
| d | 0.60 | 0.01 | 0.39 |
| e | 0.60 | 0.39 | 0.01 |
| f | 0.39 | 0.60 | 0.01 |

The ELAPOs of this invention are useful as adsorbents, catalysts, ion-exchangers, and the like in much the same fashion as aluminosilicates have been employed heretofore, although their chemical and physical properties are not necessarily similar to those observed for aluminosilicates.

ELAPO compositions are generally synthesized by hydrothermal crystallization from a reaction mixture containing reactive sources of the elements "M", aluminum and phosphorus, preferably an organic templating, i.e., structure-directing, agent, preferably a compound of an element of Group VA of the Periodic Table, and/or optionally an alkali or other metal. The reaction mixture is generally placed in a sealed pressure vessel, preferably lined with an inert plastic material such as polytetrafluoroethylene and heated, preferably under autogenous pressure at a temperature between 50°C and 250°C, and preferably between 100°C and 200°C until crystals of the ELAPO product are obtained, usually a period of from several hours to several weeks. Typical crystallization times are from about 2 hours to about 30 days with from about 2 hours to about 20 days being generally employed to obtain crystals of the ELAPO products. The product is recovered by any convenient method such as centrifugation or filtration.

In synthesizing the ELAPO compositions of the instant invention, it is preferred to employ a reaction mixture composition expressed in terms of the molar ratios as follows:

$$aR:(M_xAl_yP_z)O_2:bH_2O$$

wherein "R" is an organic templating agent; "a" is the amount of organic templating agent "R" and has a value of from zero to about 6 and is preferably an effective amount within the range of greater than zero (0) to about 6; "b" has a value of from zero (0) to about 500, preferably between about 2 and 300; "M" represents at least two elements, as above described, capable of forming tetrahedral oxide framework units, $MO_2^n$, with $AlO_2^-$ and $PO_2^+$ tetrahedral units; "n" has a value of −3, −2, −1, 0 or +1; and "x", "y" and "z" represent the mole fractions of "M", aluminum and phosphorus, respectively, "y" and "z" each have a value of at least 0.01 and "x" has a value of at least .02 with each element "M" having a mole fraction of at least 0.01. The mole fractions "x", "y" and "z" are preferably within the pentagonal compositional areas defined by points F, G, H, I, and J which is shown in Fig. 3 of the drawings, said points F, G, H, I, and J representing the following values for "x", "y" and "z":

| | Mole fraction | | |
|---|---|---|---|
| Point | x | y | z |
| F | 0.02 | 0.60 | 0.38 |
| G | 0.02 | 0.38 | 0.60 |
| H | 0.39 | 0.01 | 0.60 |
| I | 0.98 | 0.01 | 0.01 |
| J | 0.39 | 0.60 | 0.01 |

In the foregoing expression of the reaction composition, the reactants are normalized with respect to a total of (M+Al+P)=(x+y+z)=1.00 mole, whereas in many of the working examples appearing hereinafter the reaction mixtures are expressed in terms of molar oxide ratios and may be normalized to 1.00 mole of $P_2O_5$. This latter form is readily converted to the former form by routine calculations by dividing the total number of moles of "M", aluminum and phosphorus into the moles of each of "M", aluminum and phosphorus. The moles of template and water are similarly normalized by dividing the total moles of "M", aluminum and phosphorus.

In forming the reaction mixture from which the instant molecular sieves are formed the organic templating agent can be any of those heretofore proposed for use in the synthesis of conventional zeolite aluminosilicates. In general these compounds contain elements of Group VA of the Periodic Table of Elements, particularly nitrogen, phosphorus, arsenic and antimony, preferably nitrogen or phosphorus and most preferably nitrogen, which compounds also contain at least one alkyl or aryl group having from 1 to 8 carbon atoms. Particularly preferred compounds for use as templating agents are the amines, quaternary phosphonium compounds and quaternary ammonium compounds, the latter two being represented generally by the formula $R_4X^+$ wherein "X" is nitrogen or phosphorus and each R is an alkyl or aryl group containing from 1 to 8 carbon atoms. Polymeric quaternary ammonium salts such as $[(C_{14}H_{32}N_2)(OH)_2]_x$ wherein "x" has a value of at least 2 are also suitably employed. The mono-, di- and tri-amines are advantageously utilized, either alone or in combination with a quaternary ammonium compound or other templating compound. Mixtures of two or more templating agents can either produce mixtures of the desired ELAPOs or the more strongly directing templating species may control the course of the reaction with the other templating species serving primarily to establish the pH conditions of the reaction gel.

Representative templating agents include tetramethylammonium, tetraethylammonium, tetrapropylammonium or tetrabutylammonium ions; tetrapentylammonium ion; di - n - propylamine; tripropylamine; triethylamine; triethanolamine; piperidine; cyclohexylamine; 2 - methylpyridine; N,N - dimethylbenzylamine; N,N - dimethylethanolamine; choline; N,N' - dimethylpiperazine; 1,4 - diazabicyclo(2,2,2)octane; N - methyldiethanolamine, N - methylethanolamine; N - methylpiperidine; 3 -

methylpiperidine; N - methylcyclohexylamine, 3 - methylpyridine; 4 - methylpyridine; quinuclidine; N,N' - dimethyl - 1,4 - diazabicyclo(2,2,2)octane ion; di - n - butylamine, neopentylamine; di - n - pentylamine; isopropylamine; t-butylamine; ethylenediamine, pyrrolidine; and 2 - imidazolidone. Not every templating agent will direct the formation of every species of ELAPO, i.e., a single templating agent can, with proper manipulation of the reaction conditions, direct the formation of several ELAPO compositions, and a given ELAPO composition can be produced using several different templating agents.

The most suitable phosphorus source yet found for the present process is phosphoric acid, but organic phosphates such as triethyl phosphate may be satisfactory, and so also may crystalline or amorphous aluminophosphates such as the $AlPO_4$ composition of U.S.P. 4,310,440. Organo-phosphorus compounds, such as tetrabutylphosphonium bromide, do not apparently serve as reactive sources of phosphorus, but these compounds may function as templating agents. Conventional phosphorus salts such as sodium metaphosphate, may be used, at least in part, as the phosphorus source, but are not preferred.

The preferred aluminum source is either an aluminum alkoxide, such as aluminum isopropoxide, or pseudoboehmite. The crystalline or amorphous aluminophosphates which are a suitable source of phosphorus are, of course, also suitable sources of aluminum. Other sources of aluminum used in zeolite synthesis, such a gibbsite, sodium aluminate and aluminum trichloride, can be employed but are not preferred.

The elements "M" can be introduced into the reaction system in any form which permits the formation *in situ* of reactive form of the element, i.e., reactive to form the framework tetrahedral oxide unit of the element. The organic and inorganic salts, of "M" such as oxides, alkoxides, hydroxides, halides and carboxylates, may be employed including the chlorides, bromides, iodides, nitrates, sulfates, acetates, formates, ethoxides, propoxides and the like.

While not essential to the synthesis of ELAPO compositions, stirring or other moderate agitation of the reaction mixture and/or seeding the reaction mixture with seed crystals of either the ELAPO species to be produced or a topologically similar species, such as aluminophosphate, aluminosilicate or molecular sieve compositions, facilitates the crystallization procedure.

After crystallization the ELAPO product may be isolated and advantageously washed with water and dried in air. The as-synthesized ELAPO generally contains within its internal pore system at least one form of the templating agent employed in its formation. Most commonly the organic moiety is present, at least in part, as a charge-balancing cation as is generally the case with as-synthesized aluminosilicate zeolites prepared from organic-containing reaction systems. It is possible, however, that some or all of the organic moiety is an occluded molecular species in a particular ELAPO species. As a general rule the templating agent, and hence the occluded organic species, is too large to move freely through the pore system of the ELAPO product and must be removed by calcining the ELAPO at temperatures of 200°C to 700°C to thermally degrade the organic species. In a few instances the pores of the ELAPO product are sufficiently large to permit transport of the templating agent, particularly if the latter is a small molecule, and accordingly complete or partial removal thereof can be accomplished by conventional desorption procedures such as carried out in the case of zeolites. It will be understood that the term "as-synthesized" as used herein does not include the condition of the ELAPO phase wherein the organic moiety occupying the intracrystalline pore system as a result of the hydrothermal crystallization process has been reduced by post-synthesis treatment such that the value of "m" in the composition formula

$$mR:(M_xAl_yP_z)O_2$$

has a value of less than 0.02. The other symbols of the formula are as defined hereinabove. In those preparations in which an alkoxide is employed as the source of element "M", aluminum or phosphorus, the corresponding alcohol is necessarily present in the reaction mixture since it is a hydrolysis product of the alkoxide. It has not been determined whether this alcohol participates in the synthesis process as a templating agent. For the purposes of this application, however, this alcohol is arbitrarily omitted from the class of templating agents, even if it is present in the as-syntehsized ELAPO material.

Since the present ELAPO compositions are formed from $MO_2^n$, $AlO_2^-$, and $PO_2^+$ tetrahedral oxide units which, respectively, have a net charge of "n", (where "n" may be −3, −2, −1, 0 or +1), −1 and +1, the matter of cation exchangeability is considerably more complicated than in the case of zeolitic molecular sieves in which, ideally, there is a stoichiometric relationship between $AlO_2^-$ tetrahedra and charge-balancing cations. In the instant compositions, an $AlO_2^-$ tetrahedron can be balanced electrically either by association with a $PO_2^+$ tetrahedron or a simple cation such as an alkali metal cation, a proton ($H^+$), a cation of "M" present in the reaction mixture, or an organic cation derived from the templating agent. Similarly an $MO_2^n$ tetrahedron, where "n" is negative, can be balanced electrically by association with $PO_2^+$ tetrahedra, a cation of "M" present in the reaction mixture, organic cations derived from the templating agent, a simple cation such as an alkali metal cation, or other divalent or polyvalent metal cation, a proton ($H^+$), anions or cations introduced from an extraneous source. It has also been postulated that non-adjacent $AlO_2^-$ and $PO_2^+$ tetrahedral pairs can be balanced by $Na^+$ and $OH^-$ respectively [Flanigen and Grose, Molecular Sieve Zeolites-I, ACS, Washington, DC (1971)].

The ELAPO compositions of the present invention may exhibit cation-exchange capacity when analyzed using ion-exchange techniques heretofore employed with zeolitic aluminosilicates and have pore

diameters which are inherent in the lattice structure of each species and which are at least about 3Å in diameter. Ion exchange of ELAPO compositions is ordinarily possible only after the organic moiety derived from the template, present as a result of synthesis, has been removed from the pore system. Dehydration to remove water present in the as-synthesized ELAPO compositions can usually be accomplished, to some degree at least, in the usual manner without removal of the organic moiety, but the absence of the organic species greatly facilitates adsorption and desorption procedures. The ELAPO materials have various degrees of hydrothermal and thermal stability, some being quite remarkable in this regard, and function well as molecular sieve adsorbents and hydrocarbon conversion catalysts or catalyst bases.

In preparing the ELAPO compositions, it is preferred to use a stainless steel reaction vessel utilized lined with an inert plastic material, polytetrafluoroethylene, to avoid contamination of the reaction mixture. In general, the final reaction mixture from which each ELAPO composition is crystallized is prepared by forming mixtures of less than all of the reagents and thereafter incorporating into these mixtures additional reagents either singly or in the form of other intermediate mixtures of two or more reagents. In some instances the reagents admixed retain their identity in the intermediate mixture and in other cases some or all of the reagents are involved in chemical reactions to produce new reagents. The term "mixture" is applied in both cases. Further, unless otherwise specified, each intermediate mixture as well as the final reaction mixture was stirred until substantially homogeneous.

X-ray patterns of products are obtained by X-ray analysis, using either: 1) copper K-alpha radiation with Siemens Type K-805 X-ray sources with two computer interfaced Siemens D-500 X-ray powder diffractometers, available from Siemens Corporation, Cherry Hill, N. J.; or 2) standard X-ray powder diffraction techniques. When the standard technique is employed the radiation source is a high-intensity, copper target, X-ray tube operated at 50 KV and 40 mA. The diffraction pattern from the copper K-alpha radiation and graphite monochromator is suitably recorded by an X-ray spectrometer scintillation counter, pulse height analyzer and strip chart recorder. X-ray patterns are obtained using flat compressed powder samples which are scanned at 2° (2 theta) per minute, using a two second time constant.

All interplanar spacings (d) in Angstrom units are obtained from the position of the diffraction peaks expressed as 2θ where θ is the Bragg angle as observed on the strip chart. Intensities are determined from the heights of diffraction peaks after subtracting background, "$I_o$" being the intensity of the strongest line or peak, and "I" being the intensity of each of the other peaks.

As will be understood by those skilled in the art the determination of the parameter 2 theta is subject to both human and mechanical error, which in combination, can impose an uncertainty of about ±0.4° on each reported value of 2 theta. This uncertainty is, of course, also manifested in the reported values of the d-spacings, which are calculated from the 2 theta values. This imprecision is general throughout the art and is not sufficient to preclude the differentiation of the present crystalline materials from each other and from the compositions of the prior art. In some of the X-ray patterns reported, the relative intensities of the d-spacings are indicated by the notations vs, s, m, w and vw which represent very strong, strong, medium, weak and very weak, respectively.

In certain instances the purity of a synthesized product may be assessed with reference to its X-ray powder diffraction pattern. Thus, for example, if a sample may be stated to be pure, it is intended only that the X-ray pattern of the sample is free of lines attributable to crystalline impurities, not that there are no amorphous materials present.

The molecular sieves of the instant invention may be characterized by their X-ray powder diffraction patterns and such may have one of the X-ray patterns set forth in the following Tables A through V, wherein said X-ray patterns are for both the as-synthesized and calcined forms unless otherwise noted:

TABLE A (ELAPO-5)

| 2θ | d(Å) | Relative intensity |
|---|---|---|
| 7.3—7.65 | 12.1—11.56 | m—vs |
| 19.5—19.95 | 4.55—4.46 | m—s |
| 20.9—21.3 | 4.25—4.17 | m—vs |
| 22.2—22.6 | 4.00—3.93 | w—vs |
| 25.7—26.15 | 3.47—3.40 | w—m |

TABLE B (ELAPO-11)

| 2θ | d(Å) | Relative intensity |
|---|---|---|
| 9.3—9.65 | 9.51—9.17 | m—s |
| 20.2—20.6 | 4.40—4.31 | m—s |
| 20.9—21.3 | 4.25—4.17 | s—vs |
| 22.0—22.5 | 4.04—3.95 | m—s |
| 22.5—22.9 | 3.95—3.92 | m—s |
| 23.0—23.4 | 3.87—3.80 | m—vs |

TABLE C (ELAPO-14)

| 2θ | d(Å) | Relative intensity |
|---|---|---|
| 8.6—8.9 | 10.3—9.93 | vs |
| 13.0 | 6.81 | w |
| 21.9—22.2 | 4.06—4.00 | w |
| 25.4 | 3.51 | w |
| 27.5 | 3.24 | w |
| 29.7 | 3.01 | w |

TABLE D (ELAPO-16)

| 2θ | d(Å) | Relative intensity |
|---|---|---|
| 11.3—11.6 | 7.83—7.63 | m—vs |
| 18.7—18.9 | 4.75—4.70 | w—s |
| 21.9—22.3 | 4.06—3.99 | m—vs |
| 26.5—27.0 | 3.363—3.302 | w—m |
| 29.7—30.05 | 3.003—2.974 | w—m |

TABLE E (ELAPO-17)

| 2θ | d(Å) | Relative intensity |
|---|---|---|
| 7.7—7.75 | 11.5—11.4 | vs |
| 13.4 | 6.61 | s—vs |
| 15.5—15.55 | 5.72—5.70 | s |
| 19.65—19.7 | 4.52—4.51 | w—s |
| 20.5—20.6 | 4.33—4.31 | vs |
| 31.8—32.00 | 2.812—2.797 | w—s |

TABLE F (ELAPO-18)

| 2θ | d(Å) | Relative intensity |
|---|---|---|
| 9.6—9.65 | 9.21—9.16 | vs |
| 15.5—15.55 | 5.72—5.70 | m |
| 16.9—17.1 | 5.25—5.19 | m |
| 20.15—20.25 | 4.41—4.39 | m |
| 20.95—21.05 | 4.24—4.22 | m |
| 31.8—32.5 | 2.814—2.755 | m |

TABLE G (ELAPO-20)

| 2θ | d(Å) | Relative intensity |
|---|---|---|
| 13.7—14.25 | 6.46—6.22 | m—vs |
| 19.55—20.0 | 4.54—4.44 | w—s |
| 24.05—24.5 | 3.70—3.63 | m—vs |
| 34.3—35.0 | 2.614—2.564 | vw—w |
| 42.5—43.0 | 2.127—2.103 | vw—w |

TABLE H (ELAPO-31)

| 2θ | d(Å) | Relative intensity |
|---|---|---|
| 8.5—8.6 | 10.40—10.28 | m—s |
| 20.2—20.3 | 4.40—4.37 | m |
| 21.9—22.1 | 4.06—4.02 | w—m |
| 22.6—22.7 | 3.93—3.92 | vs |
| 31.7—31.8 | 2.823—2.814 | w—m |

TABLE J* (ELAPO-33)

| 2θ | d(Å) | Relative intensity |
|---|---|---|
| 9.25—9.55 | 9.56—9.26 | w—m |
| 12.5—12.9 | 7.08—6.86 | vs |
| 16.9—17.3 | 5.25—5.13 | w—m |
| 20.45—20.9 | 4.34—4.25 | w—m |
| 23.85—24.25 | 3.73—3.67 | w—m |
| 26.05—26.35 | 3.42—3.38 | w—m |
| 27.3—27.6 | 3.27—3.23 | vs |

* as-synthesized form.

TABLE K* (ELAPO-33)

| 2θ | d(Å) | Relative intensity |
|---|---|---|
| 13.15—13.4 | 6.73—6.61 | vs |
| 18.05—18.35 | 4.91—4.83 | m |
| 18.4—18.6 | 4.82—4.77 | m |
| 26.55—26.7 | 3.36—3.34 | m |
| 32.0—32.1 | 2.80—2.79 | m |

* calcined form.

TABLE L (ELAPO-34)

| 2θ | d(Å) | Relative intensity |
|---|---|---|
| 9.4—9.65 | 9.41—9.17 | s—vs |
| 15.9—16.2 | 5.57—5.47 | vw—m |
| 17.85—18.4 | 4.97—4.82 | w—s |
| 20.3—20.9 | 4.37—4.25 | m—vs |
| 24.95—25.4 | 3.57—3.51 | vw—s |
| 30.3—30.8 | 2.95—2.90 | w—s |

TABLE M (ELAPO-35)

| 2θ | d(Å) | Relative intensity |
|---|---|---|
| 10.8—11.1 | 8.19—7.97 | m |
| 17.2—17.4 | 5.16—5.10 | s—vs |
| 21.0—21.25 | 4.23—4.18 | m—s |
| 21.8—22.0 | 4.08—4.04 | vs |
| 31.8—32.2 | 2.814—2.788 | m |

TABLE N (ELAPO-36)

| 2θ | d(Å) | Relative intensity |
|---|---|---|
| 7.7—7.9 | 11.5—11.2 | vs |
| 16.2—16.6 | 5.47—5.34 | w—m |
| 18.9—19.3 | 4.70—4.60 | m—s |
| 20.6—20.8 | 4.31—4.27 | w—s |
| 21.8—22.0 | 4.08—4.04 | m |
| 22.2—22.5 | 4.00—3.95 | w—m |

TABLE O (ELAPO-37)

| 2θ | d(Å) | Relative intensity |
|---|---|---|
| 6.1—6.3 | 14.49—14.03 | vs |
| 15.5—15.7 | 5.72—5.64 | w—m |
| 18.5—18.8 | 4.80—4.72 | w—m |
| 23.5—23.7 | 3.79—3.75 | w—m |
| 26.9—27.1 | 3.31—3.29 | w—m |

TABLE P (ELAPO-39)

| 2θ | d(Å) | Relative intensity |
|---|---|---|
| 9.4—9.6 | 9.41—9.21 | w—m |
| 13.3—13.6 | 6.66—6.51 | m—vs |
| 18.0—18.4 | 4.93—4.82 | m |
| 21.2—21.5 | 4.19—4.13 | m—s |
| 22.5—23.0 | 3.95—3.87 | s—vs |
| 30.2—30.5 | 2.96—2.93 | w—m |

TABLE Q (ELAPO-40)

| 2θ | d(Å) | Relative intensity |
|---|---|---|
| 7.5—7.7 | 11.79—11.48 | vw—m |
| 8.0—8.1 | 11.05—10.94 | s—vs |
| 12.4—12.5 | 7.14—7.08 | w—vs |
| 13.5—13.8 | 6.51—6.42 | m—s |
| 14.0—14.1 | 6.33—6.28 | w—m |
| 27.8—28.0 | 3.209—3.187 | w—m |

TABLE R (ELAPO-41)

| 2θ | d(Å) | Relative intensity |
|---|---|---|
| 13.6—13.8 | 6.51—6.42 | w—m |
| 20.5—20.6 | 4.33—4.31 | w—m |
| 21.1—21.3 | 4.21—4.17 | vs |
| 22.1—22.3 | 4.02—3.99 | m—s |
| 22.8—23.0 | 3.90—3.86 | m |
| 23.1—23.4 | 3.82—3.80 | w—m |
| 25.5—25.9 | 3.493—3.440 | w—m |

TABLE S (ELAPO-42)

| 2θ | d(Å) | Relative intensity |
|---|---|---|
| 7.15—7.4 | 12.35—11.95 | m—vs |
| 12.5—12.7 | 7.08—6.97 | m—s |
| 21.75—21.9 | 4.09—4.06 | m—s |
| 24.1—24.25 | 3.69—3.67 | vs |
| 27.25—27.4 | 3.273—3.255 | s |
| 30.05—30.25 | 2.974—2.955 | m—s |

TABLE T (ELAPO-44)

| 2θ | d(Å) | Relative intensity |
|---|---|---|
| 9.4—9.55 | 9.41—9.26 | vs |
| 13.0—13.1 | 6.81—6.76 | w—m |
| 16.0—16.2 | 5.54—5.47 | w—m |
| 20.6—20.85 | 4.31—4.26 | s—vs |
| 24.3—24.4 | 3.66—3.65 | w—vs |
| 30.7—30.95 | 2.912—2.889 | w—s |

TABLE U (ELAPO-46)

| 2θ | d(Å) | Relative intensity |
|---|---|---|
| 7.2—8.1 | 12.3—10.9 | vs |
| 21.2—21.8 | 4.19—4.08 | w—m |
| 22.5—23.0 | 3.95—3.87 | vw—m |
| 26.6—27.2 | 3.351—3.278 | vw—w |
| 28.5—29.0 | 3.132—3.079 | vw—w |

TABLE V (ELAPO-47)

| 2θ | d(Å) | Relative intensity |
|---|---|---|
| 9.4 | 9.41 | vs |
| 15.9—16.0 | 5.57—5.54 | w—m |
| 20.5—20.6 | 4.33—4.31 | s |
| 24.5—24.7 | 3.63—3.60 | w |
| 25.8—25.9 | 3.45—3.44 | w |
| 30.4—30.5 | 2.940—2.931 | w |

The following examples are provided to further illustrate the invention and are not intended to be limiting thereof:

Example 1 (preparation of BeMgAPO-5)

a) BeMgAPO-5 is prepared from a reaction mixture having a composition, expressed in terms of the molar oxide ratios of the components of the reaction mixture, of:

$$1.0\text{—}2.0\ TPA:0.05\text{—}0.2\ (M)_2O_q:0.5\text{—}1.0\ Al_2O_3:0.5\text{—}1.0\ P_2O_5:40\text{—}100\ H_2O$$

where "TPA" denotes tripropylamine and "q' denotes the oxidation state of "M" (beryllium and magnesium).

The reaction mixture is digested by placing the reaction mixture in a sealed stainless steel pressure vessel and heating it at an effective temperature and for an effective time until crystals of the BeMgAPO-5 product are obtained. Solids are recovered by filtration, washed with water and dried in air at room temperature.

The BeMgAPO-5 product's chemical analysis shows the BeMgAPO-5 product contains beryllium, magnesium, aluminum and phosphorus in amounts within the pentagonal compositional area defined by points A, B, C, D and E of Fig. 1.

The X-ray powder diffraction pattern of an BeMgAPO-5 product is characterized by the following data:

| 2θ | d(Å) | Relative intensity |
|---|---|---|
| 7.3—7.65 | 12.1—11.56 | m—vs |
| 19.5—19.95 | 4.55—4.46 | m—s |
| 20.9—21.3 | 4.25—4.17 | m—vs |
| 22.2—22.6 | 4.00—3.93 | w—vs |
| 25.7—26.15 | 3.47—3.40 | w—m |

b) The X-ray powder diffraction pattern for a calcined BeMgAPO-5 is also characterized by the X-ray pattern of part a).

c) When the calcined BeMgAPO-5 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic diameter (Å) | Pressure (1.33 mbar) | Temp., °C | Wt. % adsorbed* |
|---|---|---|---|---|
| $O_2$ | 3.46 | 100 | −183 | 7 |
| $O_2$ | 3.46 | 750 | −183 | 10 |
| Neopentane | 6.2 | 700 | 24 | 4 |
| $H_2O$ | 2.65 | 4.3 | 24 | 4 |
| $H_2O$ | 2.65 | 20.0 | 24 | 12 |

* Typical amount adsorbed.

The pore diameter of BeMgAPO-5 is greater than 6.2Å.

Example 2 (preparation of CrMgAPO-11)

a) CrMgAPO-11 is prepared from a reaction mixture having a composition, expressed in terms of the molar oxide ratios of the components of the reaction mixture, of:

$$1.0—2.0\ DPA:0.05—0.2\ (M)_2O_q:0.5—1.0\ Al_2O_3:0.5—1.0\ P_2O_5:40—100\ H_2O$$

where "DPA" denotes di-n-propylamine and "q" denotes the oxidation state of "M" (chromium and magnesium).

The reaction mixture is digested by placing the reaction mixture in a sealed stainless steel pressure vessel and heating it at an effective temperature and for an effective time until crystals of the CrMgAPO-11 product are obtained. Solids are then recovered by filtration, washed with water and dried in air at room temperature.

The CrMgAPO-11 product's chemical analysis shows the CrMgAPO-11 product contains chromium, magnesium, aluminum and phosphorus in amounts within the pentagonal compositional area defined by points A, B, C, D and E of Fig. 1.

The X-ray powder diffraction pattern of an CrMgAPO-11 product is characterized by the following data:

| 2θ | d(Å) | Relative intensity |
|---|---|---|
| 9.3—9.65 | 9.51—9.17 | m—s |
| 20.2—20.6 | 4.40—4.31 | m—s |
| 20.9—21.3 | 4.25—4.17 | s—vs |
| 22.0—22.5 | 4.04—3.95 | m—s |
| 22.5—22.9 | 3.95—3.92 | m—s |
| 23.0—23.4 | 3.87—3.80 | m—vs |

b) The X-ray powder diffraction pattern for a calcined CrMgAPO-11 is also characterized by the X-ray pattern of part a).

c) When the calcined CrMgAPO-11 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic diameter (Å) | Pressure (1.33 mbar) | Temp., °C | Wt. % adsorbed* |
|---|---|---|---|---|
| $O_2$ | 3.46 | 100 | −183 | 5 |
| $O_2$ | 3.46 | 750 | −183 | 6 |
| Cyclohexane | 6.0 | 90 | 24 | 4 |
| $H_2O$ | 2.65 | 4.3 | 24 | 6 |
| $H_2O$ | 2.65 | 20 | 24 | 8 |

* Typical amount adsorbed.

The pore diameter of CrMgAPO-11 is about 6Å.

Example 3 (preparation of MgCoVAPO-17)

a) MgCoVAPO-17 is prepared from a reaction mixture having a composition, expressed in terms of the molar oxide ratios of the components of the reaction mixture, of:

$$1.0—2.0\ QN:0.05—0.2\ (M)_2O_q:0.5—1.0\ Al_2O_3:0.5—1.0\ P_2O_5:40—100\ H_2O$$

where "QN" denotes quinuclidine and "q" denotes the oxidation state of "M" (magnesium, cobalt and vanadium).

The reaction mixture is digested by placing the reaction mixture in a sealed stainless steel pressure vessel and heating it at an effective temperature and for an effective time until crystals of the MgCoVAPO-17 product are obtained. Solids are then recovered by filtration, washed with water and dried in air at room temperature.

The MgCoVAPO-17 product's chemical analysis shows the MgCoVAPO-17 product contains magnesium, cobalt, vanadium, aluminum and phosphorus in amounts within the pentagonal compositional area defined by points A, B, C, D and E of Fig. 1.

The X-ray powder diffraction pattern of an MgCoVAPO-17 product is characterized by the following data:

| 2θ | d(Å) | Relative intensity |
|---|---|---|
| 7.7—7.75 | 11.5—11.4 | vs |
| 13.4 | 6.61 | s—vs |
| 15.5—15.55 | 5.72—5.70 | s |
| 19.65—19.7 | 4.52—4.51 | w—s |
| 20.5—20.6 | 4.33—4.31 | vs |
| 31.8—32.00 | 2.812—2.797 | w—s |

b) The X-ray powder diffraction pattern for a calcined MgCoVAPO-17 is also characterized by the X-ray pattern of part a).

c) When the calcined MgCoVAPO-17 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic diameter (Å) | Pressure (1.33 mbar) | Temp., °C | Wt. % adsorbed* |
|---|---|---|---|---|
| $O_2$ | 3.46 | 100 | −183 | 10 |
| $O_2$ | 3.46 | 750 | −183 | 12 |
| n-Butane | 4.3 | 100 | 24 | 4 |
| $H_2O$ | 2.65 | 4.3 | 24 | 13 |
| $H_2O$ | 2.65 | 20 | 24 | 14 |

* Typical amount adsorbed.

The pore diameter of MgCoVAPO-17 is about 4.3Å.

Example 4 (preparation of FeAsAPO-31)

a) FeAsAPO-31 is prepared from a reaction mixture having a composition, expressed in terms of the molar oxide ratios of the components of the reaction mixture, of:

$$1.0\text{—}2.0\ \text{DPA}:0.05\text{—}0.2\ (M)_2O_q:0.5\text{—}1.0\ Al_2O_3:0.5\text{—}1.0\ P_2O_5:40\text{—}100\ H_2O$$

wherein "DPA" denotes di-n-propylamine and "q" denotes the oxidation state of "M" (iron and arsenic).

The reaction mixture is seeded with crystals of $AlPO_4$-31 (U.S. Patent No. 4,310,440) and digested by placing the reaction mixture in a sealed stainless steel pressure vessel and heating it at an effective temperature and for an effective time until crystals of the FeAsAPO-31 product are obtained. Solids are then recovered by filtration, washed with water and dried in air at room temperature.

The FeAsAPO-31 product's chemical analysis shows the FeAsAPO-31 product contains iron, arsenic, aluminum and phosphorus in amounts within the pentagonal compositional area defined by points A, B, C, D and E of Fig. 1.

The X-ray powder diffraction pattern of an FeAsAPO-31 product is characterized by the following data:

| 2θ | d(Å) | Relative intensity |
|---|---|---|
| 8.5—8.6 | 10.40—10.28 | m—s |
| 20.2—20.3 | 4.40—4.37 | m |
| 21.9—22.1 | 4.06—4.02 | w—m |
| 22.6—22.7 | 3.93—3.92 | vs |
| 31.7—31.8 | 2.823—2.814 | w—m |

b) The X-ray powder diffraction pattern for a calcined FeAsAPO-31 is also characterized by the X-ray pattern of part a).

c) When the calcined FeAsAPO-31 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic diameter (Å) | Pressure (1.33 mbar) | Temp., °C | Wt. % adsorbed* |
|---|---|---|---|---|
| $O_2$ | 3.46 | 100 | −183 | 4 |
| $O_2$ | 3.46 | 750 | −183 | 6 |
| Cyclohexane | 6.0 | 90 | 24 | 3 |
| Neopentane | 6.2 | 700 | 24 | 3 |
| $H_2O$ | 2.65 | 4.3 | 24 | 3 |
| $H_2O$ | 2.65 | 20 | 24 | 10 |

* Typical amount adsorbed.

The pore diameter of FeAsAPO-31 is greater than about 6.2Å.

Example 5 (preparation of GeMgAPO-34)

a) GeMgAPO-34 is prepared from a reaction mixture having a composition, expressed in terms of the molar oxide ratios of the components of the reaction mixture, of:

$$1.0—2.0\ TEAOH:0.05—0.2\ (M)_2O_q:0.5—1.0\ Al_2O_3:0.5—1.0\ P_2O_5:40—100\ H_2O$$

where "TEAOH" denotes tetraethylammonium hydroxide and "q" denotes the oxidation state of "M" (germanium and magnesium).

The reaction mixture is digested by placing the reaction mixture in a sealed stainless steel pressure vessel and heating it at an effective temperature and for an effective time until crystals of the GeMgAPO-34 product are obtained. The solids are recovered by filtration, washed with water and dried in air at room temperature.

The GeMgAPO-34 product's chemical analysis shows the GeMgAPO-34 product contains germanium, magnesium, aluminum and phosphorus in amounts within the pentagonal compositional area defined by points A, B, C, D and E of Fig. 1.

The X-ray powder diffraction pattern of a GeMgAPO-34 product is characterized by the following data:

| 2θ | d(Å) | Relative intensity |
|---|---|---|
| 9.4—9.65 | 9.41—9.17 | s—vs |
| 15.9—16.2 | 5.57—5.47 | vw—m |
| 17.85—18.4 | 4.97—4.82 | w—s |
| 20.3—20.9 | 4.37—4.25 | m—vs |
| 24.95—25.4 | 3.57—3.51 | vw—s |
| 30.3—30.8 | 2.95—2.90 | w—s |

b) The X-ray powder diffraction pattern for a calcined GeMgAPO-34 is also characterized by the X-ray pattern of part a).

c) When the calcined GeMgAPO-34 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic diameter (Å) | Pressure (1.33 mbar) | Temp., °C | Wt. % adsorbed* |
|---|---|---|---|---|
| $O_2$ | 3.46 | 100 | −183 | 13 |
| $O_2$ | 3.46 | 750 | −183 | 18 |
| n-Hexane | 4.3 | 100 | 24 | 6 |
| $H_2O$ | 2.65 | 4.3 | 24 | 15 |
| $H_2O$ | 2.65 | 20 | 24 | 21 |

* Typical amount adsorbed.

The pore diameter of GeMgAPO-34 is about 4.3Å.

Example 6 (preparation of GeMgCoAPO-44)

a) GeMgCoAPO-44 may be prepared from a reaction mixture having a composition, expressed in terms of the molar oxide ratios of the components of the reaction mixture, of:

$$1.0—2.0\ CHA:0.05—0.2\ (M)_2O_q:0.5—1.0\ Al_2O_3:0.5—1.0\ P_2O_5:40—100\ H_2O$$

where "CHA" denotes cyclohexylamine and "q" denotes the oxidation state of "M" (germanium, cobalt and magnesium).

The reaction mixture is digested by placing the reaction mixture in a sealed stainless steel pressure vessel and heating it at an effective temperature and for an effective time until crystals of the GeMgCoAPO-44 product are obtained. Solids are then recovered by filtration, washed with water and dried in air at room temperature.

The GeMgCoAPO-44 product's chemical analysis shows the GeMgCoAPO-44 product contains germanium, magnesium, cobalt, aluminum and phosphorus in amounts within the pentagonal compositional area defined by points, A, B, C, D and E of Fig. 1.

The X-ray powder diffraction pattern of an GeMgCoAPO-44 product is characterized by the following data:

| 2θ | d(Å) | Relative intensity |
|---|---|---|
| 9.4—9.55 | 9.41—9.26 | vs |
| 13.0—13.1 | 6.81—6.76 | w—m |
| 16.0—16.2 | 5.54—5.47 | w—m |
| 20.6—20.85 | 4.31—4.26 | s—vs |
| 24.3—24.4 | 3.66—3.65 | w—vs |
| 30.7—30.95 | 2.912—2.889 | w—s |

b) When the calcined GeMgCoAPO-44 is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic diameter (Å) | Pressure (1.33 mbar) | Temp., °C | Wt. % adsorbed* |
|---|---|---|---|---|
| $O_2$ | 3.46 | 100 | −183 | 13 |
| $O_2$ | 3.46 | 750 | −183 | 16 |
| n-Hexane | 4.3 | 100 | 24 | 2 |
| $H_2O$ | 2.65 | 4.3 | 24 | 15 |
| $H_2O$ | 2.65 | 20 | 24 | 17 |

* Typical amount adsorbed.

The pore diameter of GeMgCoAPO-44 is about 4.3Å.

Process applications

The ELAPO compositions of the present invention are, in general, hydrophilic and adsorb water preferentially over common hydrocarbon molecules such as paraffins, olefins and aromatic species, e.g., benzene, xylenes and cumene. Thus, the present ELAPO compositions as a class are useful in a process for separating molecular species from admixture with molecular species having a lesser degree of polarity which comprises contacting said mixture of molecular species with an ELAPO molecular sieve having pore diameter large enough to adsorb at least one of the more polar molecular species, said molecular sieve being at least partially activated whereby molecules of the more polar molecular species are selectively adsorbed into the intracrystalline pore system thereof.

The ELAPO compositions are as well useful in a process for separating a mixture of molecular species having different kinetic diameters which comprises contacting said mixture with an ELAPO molecular sieve having pore diameters large enough to adsorb at least one but not all molecular species of said mixture, said molecular sieve being at least partially activated whereby at least some molecules whose kinetic diameters are sufficiently small can enter the intracrystalline pore system thereof.

ELAPO compositions are useful as desiccants in such adsorption separation/purification processes as natural gas drying, cracked gas drying. Water is also preferentially adsorbed over the so-called permanent gases such as carbon dioxide, nitrogen, oxygen and hydrogen. These ELAPOs are therefore suitably employed in the drying of reformer hydrogen streams and in the drying of oxygen, nitrogen or air prior to liquefaction.

The present ELAPO compositions also exhibit novel surface selectivity characteristics which render them useful as catalyst or catalyst bases in a number of hydrocarbon conversion and oxidative combustion reactions. They can be impregnated or otherwise loaded with catalytically active metals by methods well known in the art and used, for example, in fabricating catalyst compositions having silica or alumina bases. Of the general class, those species having pores larger than about 4Å are preferred for catalytic applications.

Among the hydrocarbon conversion reactions catalyzed by ELAPO compositions are cracking, hydrocracking, alkylation for both the aromatic and isoparaffin types, isomerization including xylene

isomerization, polymerization, reforming, hydrogenation, dehydrogenation, transalkylation, dealkylation, hydrodecyclization and dehydrocyclization.

Using ELAPO catalyst compositions which contain a hydrogenation promoter such as platinum or palladium, heavy petroleum residual stocks, cyclic stocks and other hydrocrackable charge stocks, can be hydrocracked at temperatures in the range of 204.4°C to 440.6°C (400°F to 825°F) using molar ratios of hydrogen to hydrocarbon in the range of between 2 and 80, pressures between 1.7 and 242.1 bar (10 and 3500 p.s.i.g.), and a liquid hourly space velocity (LHSV) of from 0.1 to 20, preferably 1.0 to 10.

The ELAPO catalyst compositions employed in hydrocracking are also suitable for use in reforming processes in which the hydrocarbon feedstocks contact the catalyst at temperatures of from about 371.1°C to 537.8°C (700°F to 1000°F), hydrogen pressures of from 7.9 to 35.46 bar (100 to 500 p.s.i.g.), LHSV values in the range of 0.1 to 10 and hydrogen to hydrocarbon molar ratios in the range of 1 to 20, preferably between 4 and 12.

These same catalysts, i.e. those containing hydrogenation promoters, are also useful in hydroisomerizations processes in which feedstocks such as normal paraffins are converted to saturated branched chain isomers. Hydroisomerization is carried out at a temperature of from about 93.3°C to 315.5°C (200°F to 600°F), preferably 148.9°C to 287.8°C (300°F to 550°F) with an LHSV value of from about 0.2 to 1.0. Hydrogen is supplied the reactor in admixture with the hydrocarbon feedstock in molar proportions (hydrogen/hydrocarbon) of between 1 and 5.

At somewhat higher temperatures, i.e. from about 343.3°C to 537.8°C (650°F to 1000°F), preferably 454.4°C to 510.0°C (850°F to 950°F) and usually at somewhat lower pressures within the range of about 2.04 to 4.46 bar (15 to 50 p.s.i.g.), the same catalyst compositions are used to hydroisomerize normal paraffins. Preferably the paraffin feedstock comprises normal paraffins having a carbon number range of $C_7$—$C_{20}$. Contact time between the feedstock and the catalyst is generally relatively short to avoid undesirable side reactions such as olefin polymerization and paraffin cracking. LHSV values in the range of 0.1 to 10, preferably 1.0 to 6.0 are suitable.

The unique crystal structure of the present ELAPO catalysts and their availability in a form totally void of alkali metal content favor their use in the conversion of alkylaromatic compounds, particularly the catalytic disproportionation of toluene, ethylene, trimethyl benzenes, tetramethyl benzenes and the like. In the disproportionation process, isomerization and transalkylation can also occur. Group VIII noble metal adjuvants alone or in conjunction with Group VI-B metals such as tungsten, molybdenum and chromium are preferably included in the catalyst composition in amounts of from about 3 to 15 weight-% of the overall composition. Extraneous hydrogen can, but need not, be present in the reaction zone which is maintained at a temperature of from about 204.4°C to 398.9°C (400 to 750°F), pressures in the range of 7.9 to 138.8 bar (100 to 2000 p.s.i.g.) and LHSV values in the range of 0.1 to 15.

Catalytic cracking processes are preferably carried out with ELAPO compositions using feedstocks such as gas oils, heavy naphthas, deasphalted crude oil residua, etc., with gasoline being the principal desired product. Temperature conditions of 454°C to 593.3°C (850 to 1100°F), LHSV values of 0.5 to 10 and pressure conditions of from about 1.01 to 4.46 bar (0 to 50 p.s.i.g.) are suitable.

Dehydrocyclization reactions employing paraffinic hydrocarbon feedstocks, preferably normal paraffins having more than 6 carbon atoms, to form benzene, xylenes, toluene and the like are carried out using essentially the same reaction conditions as for catalytic cracking. For these reactions it is preferred to use the ELAPO catalyst in conjunction with a Group VIII non-noble metal cation such as cobalt and nickel.

In catalytic dealkylation wherein it is desired to cleave paraffinic side chains from aromatic nuclei without substantially hydrogenating the ring structure, relatively high temperatures in the range of about 426.7°C—537.8°C (800°F—1000°F) are employed at moderate hydrogen pressures of about 21.7—69.9 bar (300—1000 p.s.i.g.), other conditions being similar to those described above for catalytic hydrocracking. Preferred catalysts are of the same type described above in connection with catalytic dehydrocyclization. Particularly desirable dealkylation reactions contemplated herein include the conversion of methylnaphthalene to naphthalene and toluene and/or xylenes to benzene.

In catalytic hydrofining, the primary objective is to promote the selective hydrodecomposition of organic sulfur and/or nitrogen compounds in the feed, without substantially affecting hydrocarbon molecules therein. For this purpose it is preferred to employ the same general conditions described above for catalytic hydrocracking, and catalysts of the same general nature described in connection with dehydrocyclization operations. Feedstocks include gasoline fractions, kerosenes, jet fuel fractions, diesel fractions, light and heavy gas oils, deasphalted crude oil residua and the like any of which may contain up to about 5 weight percent of sulfur and up to about 3 weight percent of nitrogen.

Similar conditions can be employed to effect hydrofining, i.e., denitrogenation and desulfurization, of hydrocarbon feeds containing substantial proportions of organonitrogen and organosulfur compounds. It is generally recognized that the presence of substantial amounts of such constituents markedly inhibits the activity of hydrocracking catalysts. Consequently, it is necessary to operate at more extreme conditions when it is desired to obtain the same degree of hydrocracking conversion per pass on a relatively nitrogenous feed than are required with a feed containing less organonitrogen compounds. Consequently, the conditions under which denitrogenation, desulfurization and/or hydrocracking can be most expeditiously accomplished in any given situation are necessarily determined in view of the characteristics of the feedstocks in particular the concentration of organonitrogen compounds in the feedstock. As a result

of the effect of organonitrogen compounds on the hydrocracking activity of these compositions it is not at all unlikely that the conditions most suitable for denitrogenation of a given feedstock having a relatively high organonitrogen content with minimal hydrocracking, e.g., less than 20 volume percent of fresh feed per pass, might be the same as those preferred for hydrocracking another feedstock having a lower concentration of hydrocracking inhibiting constituents e.g., organonitrogen compounds. Consequently, it has become the practice in this art to establish the conditions under which a certain feed is to be contacted on the basis of preliminary screening tests with the specific catalyst and feedstock.

Isomerization reactions are carried out under conditions similar to those described above for reforming, using somewhat more acidic catalysts. Olefins are preferably isomerized at temperatures of 260.0°C—482.2°C (500°F—900°F), while paraffin naphthenes and alkyl aromatics are isomerized at temperatures of 371.1°C—537.8°C (700°F—1000°F). Particularly desirable isomerization reactions contemplated herein include the conversion of n-heptene and/or n-octane to isoheptanes, iso-octanes, butane to iso-butane, methylcyclopentane to cyclohexane, meta-xylene and/or ortho-xylene to paraxylene, 1-butene to 2-butene and/or isobutene, n-hexene to isohexene, cyclohexene to methylcyclopentene etc. The preferred form of the catalyst is a combination of the ELAPO with polyvalent metal compounds (such as sulfides) of metals of Group II-A, Group II-B and rare earth metals. For alkylation and dealkylation processes the ELAPO compositions having pores of at least 5Å are preferred. When employed for dealkylation of alkyl, aromatics, the temperature is usually at least 176.7°C (350°F) and ranges up to a temperature at which substantial cracking of the feedstock or conversion products occurs, generally up to about 371.1°C (700°F). The temperature is preferably at least 232.2°C (450°F) and not greater than the critical temperature of the compound undergoing dealkylation. Pressure conditions are applied to retain at least the aromatic feed in the liquid state. For alkylation the temperature can be as low as 121.1°C (250°F) but is preferably at least 176.7°C (350°F). In the alkylation of benzene, toluene and xylene, the preferred alkylating agents are olefins such as ethylene and propylene.

**Claims**

1. Crystalline molecular sieves having three-dimensional microporous framework structures of $AlO_2^-$, $PO_2^+$ and $MO_2^n$ tetrahedral units with charge n, wherein "n" may be −3, −2, −1, 0 or +1 having an empirical chemical composition on an anhydrous basis expressed by the formula:

$$mR:(M_xAl_yP_z)O_2$$

wherein "R" represents at least one organic templating agent present in the intracrystalline pore system; "m" represents the molar amount of "R" present per mole of $(M_xAl_yP_z)O_2$ and has a value of zero to about 0.3; "M" represents at least two elements capable of forming framework tetrahedral oxide units and comprises at least one element selected from the group consisting of arsenic, beryllium, boron, chromium, gallium, germanium, lithium and vanadium and at least one element selected from the group consisting of cobalt, magnesium, manganese, iron, titanium and zinc; "x", "y" and "z" represent the mole fractions of "M", aluminum and phosphorus respectively, and are within a pentagonal compositional area defined by points A, B, C, D and E, said points representing the following values for "x", "y" and "z":

| | Mole fraction | | |
|---|---|---|---|
| Point | x | y | z |
| A | 0.02 | 0.60 | 0.38 |
| B | 0.02 | 0.38 | 0.60 |
| C | 0.39 | 0.01 | 0.60 |
| D | 0.98 | 0.01 | 0.01 |
| E | 0.39 | 0.60 | 0.01 |

2. The crystalline molecular sieves of claim 1 wherein "x", "y" and "z" are within a hexagonal compositional area defined by points a, b, c, d, e and f, said points representing the following values for "x", "y" and "z":

| | Mole fraction | | |
|---|---|---|---|
| Point | x | y | z |
| a | 0.02 | 0.60 | 0.38 |
| b | 0.02 | 0.38 | 0.60 |
| c | 0.39 | 0.01 | 0.60 |
| d | 0.60 | 0.01 | 0.39 |
| e | 0.60 | 0.39 | 0.01 |
| f | 0.39 | 0.60 | 0.01 |

3. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 7.3—7.65 | 12.1—11.56 | m—vs |
| 19.5—19.95 | 4.55—4.46 | m—s |
| 20.9—21.3 | 4.25—4.17 | m—vs |
| 22.2—22.6 | 4.00—3.93 | w—vs |
| 25.7—26.15 | 3.47—3.40 | w—m |

4. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 9.3—9.65 | 9.51—9.17 | m—s |
| 20.2—20.6 | 4.40—4.31 | m—s |
| 20.9—21.3 | 4.25—4.17 | s—vs |
| 22.0—22.5 | 4.04—3.95 | m—s |
| 22.5—22.9 | 3.95—3.92 | m—s |
| 23.0—23.4 | 3.87—3.80 | m—vs |

5. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 8.6—8.9 | 10.3—9.93 | vs |
| 13.0 | 6.81 | w |
| 21.9—22.2 | 4.06—4.00 | w |
| 25.4 | 3.51 | w |
| 27.5 | 3.24 | w |
| 29.7 | 3.01 | w |

6. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 11.3—11.6 | 7.83—7.63 | m—vs |
| 18.7—18.9 | 4.75—4.70 | w—s |
| 21.9—22.3 | 4.06—3.99 | m—vs |
| 26.5—27.0 | 3.363—3.302 | w—m |
| 29.7—30.05 | 3.008—2.974 | w—m |

7. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 7.7—7.75 | 11.5—11.4 | vs |
| 13.4 | 6.61 | s—vs |
| 15.5—15.55 | 5.72—5.70 | s |
| 19.65—19.7 | 4.52—4.51 | w—s |
| 20.5—20.6 | 4.33—4.31 | vs |
| 31.8—32.00 | 2.812—2.797 | w—s |

8. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 9.6—9.65 | 9.21—9.16 | vs |
| 15.5—15.55 | 5.72—5.70 | m |
| 16.9—17.1 | 5.25—5.19 | m |
| 20.15—20.25 | 4.41—4.39 | m |
| 20.95—21.05 | 4.24—4.22 | m |
| 31.8—32.5 | 2.814—2.755 | m |

9. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 13.7—14.25 | 6.46—6.22 | m—vs |
| 19.55—20.0 | 4.54—4.44 | w—s |
| 24.05—24.5 | 3.70—3.63 | m—vs |
| 34.3—35.0 | 2.614—2.564 | vw—w |
| 42.5—43.0 | 2.127—2.103 | vw—w |

10. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 8.5—8.6 | 10.40—10.28 | m—s |
| 20.2—20.3 | 4.40—4.37 | m |
| 21.9—22.1 | 4.06—4.02 | w—m |
| 22.6—22.7 | 3.93—3.92 | vs |
| 31.7—31.8 | 2.823—2.814 | w—m |

11. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 9.25—9.55 | 9.56—9.26 | w—m |
| 12.5—12.9 | 7.08—6.86 | vs |
| 16.9—17.3 | 5.25—5.13 | w—m |
| 20.45—20.9 | 4.34—4.25 | w—m |
| 23.85—24.25 | 3.73—3.67 | w—m |
| 26.05—26.35 | 3:42—3.38 | w—m |
| 27.3—27.6 | 3.27—3.23 | vs |

12. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 13.15—13.4 | 6.73—6.61 | vs |
| 18.05—18.35 | 4.91—4.83 | m |
| 18.4—18.6 | 4.82—4.77 | m |
| 26.55—26.7 | 3.36—3.34 | m |
| 32.0—32.1 | 2.80—2.79 | m |

13. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 9.4—9.65 | 9.41—9.17 | s—vs |
| 15.9—16.2 | 5.57—5.47 | vw—m |
| 17.85—18.4 | 4.97—4.82 | w—s |
| 20.3—20.9 | 4.37—4.25 | m—vs |
| 24.95—25.4 | 3.57—3.51 | vw—s |
| 30.3—30.8 | 2.95—2.90 | w—s |

14. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 10.8—11.1 | 8.19—7.97 | m |
| 17.2—17.4 | 5.16—5.10 | s—vs |
| 21.0—21.25 | 4.23—4.18 | m—s |
| 21.8—22.0 | 4.08—4.04 | vs |
| 31.8—32.2 | 2.814—2.788 | m |

15. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 7.7—7.9 | 11.5—11.2 | vs |
| 16.2—16.6 | 5.47—5.34 | w—m |
| 18.9—19.3 | 4.70—4.60 | m—s |
| 20.6—20.8 | 4.31—4.27 | w—s |
| 21.8—22.0 | 4.08—4.04 | m |
| 22.2—22.5 | 4.00—3.95 | w—m |

16. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 6.1—6.3 | 14.49—14.03 | vs |
| 15.5—15.7 | 5.72—5.64 | w—m |
| 18.5—18.8 | 4.80—4.72 | w—m |
| 23.5—23.7 | 3.79—3.75 | w—m |
| 26.9—27.1 | 3.31—3.29 | w—m |

17. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 9.4—9.6 | 9.41—9.21 | w—m |
| 13.3—13.6 | 6.66—6.51 | m—vs |
| 18.0—18.4 | 4.93—4.82 | m |
| 21.2—21.5 | 4.19—4.13 | m—s |
| 22.5—23.0 | 3.95—3.87 | s—vs |
| 30.2—30.5 | 2.96—2.93 | w—m |

18. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 7.5—7.7 | 11.79—11.48 | vw—m |
| 8.0—8.1 | 11.05—10.94 | s—vs |
| 12.4—12.5 | 7.14—7.08 | w—vs |
| 13.6—13.8 | 6.51—6.42 | m—s |
| 14.0—14.1 | 6.33—6.28 | w—m |
| 27.8—28.0 | 3.209—3.187 | w—m |

19. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 13.6—13.8 | 6.51—6.42 | w—m |
| 20.5—20.6 | 4.33—4.31 | w—m |
| 21.1—21.3 | 4.21—4.17 | vs |
| 22.1—22.3 | 4.02—3.99 | m—s |
| 22.8—23.0 | 3.90—3.86 | m |
| 23.1—23.4 | 3.82—3.80 | w—m |
| 25.5—25.9 | 3.493—3.440 | w—m |

20. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 7.15—7.4 | 12.36—11.95 | m—vs |
| 12.5—12.7 | 7.08—6.97 | m—s |
| 21.75—21.9 | 4.09—4.06 | m—s |
| 24.1—24.25 | 3.69—3.67 | vs |
| 27.25—27.4 | 3.273—3.255 | s |
| 30.05—30.25 | 2.974—2.955 | m—s |

21. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 9.4—9.55 | 9.41—9.26 | vs |
| 13.0—13.1 | 6.81—6.76 | w—m |
| 16.0—16.2 | 5.54—5.47 | w—m |
| 20.6—20.85 | 4.31—4.26 | s—vs |
| 24.3—24.4 | 3.66—3.65 | w—vs |
| 30.7—30.95 | 2.912—2.889 | w—s |

22. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 7.2—8.1 | 12.3—10.9 | vs |
| 21.2—21.8 | 4.19—4.08 | w—m |
| 22.5—23.0 | 3.95—3.87 | vw—m |
| 26.6—27.2 | 3.351—3.278 | vw—w |
| 28.5—29.0 | 3.132—3.079 | vw—w |

23. The crystalline molecular sieves of claims 1 or 2 having a characteristic X-ray powder diffraction pattern which contains at least the d-spacings set forth in the following Table:

| 2θ | d(Å) | Relative intensity |
|---|---|---|
| 9.4 | 9.41 | vs |
| 15.9—16.0 | 5.57—5.54 | w—m |
| 20.5—20.6 | 4.33—4.31 | s |
| 24.5—24.7 | 3.63—3.60 | w |
| 25.8—25.9 | 3.45—3.44 | w |
| 30.4—30.5 | 2.940—2.931 | w |

24. The molecular sieve according to claims 1 or 2 comprising a three-dimensional microporous framework structure of $MO_2^n$, $AlO_2^-$ and $PO_2^+$ tetrahedral units where "n" is −2, −1, 0 or +1.

25. Molecular sieves according to claim 1 wherein "M" is beryllium and magnesium.

26. Molecular sieves according to claim 1 wherein "M" is at least one of the group consisting of arsenic, germanium, chromium and vanadium and at least one of the group consisting of manganese, magnesium, iron and zinc.

27. The molecular sieves according to claim 1 wherein "M" is at least one of boron and gallium and at least one of the group consisting of iron, magnesium, manganese and zinc.

28. The molecular sieves according to claim 1 wherein "M" is magnesium and germanium.

29. The molecular sieves of claim 1 wherein "M" is at least one element selected from the group consisting of beryllium and germanium and at least one element selected from the group consisting of magnesium, manganese, iron and zinc.

30. The molecular sieves of claim 26 wherein "M" is chromium and magnesium.

31. The molecular sieves of claim 26 wherein "M" is iron and arsenic.

32. Process for preparing the crystalline molecular sieves of claim 1 having three-dimensional microporous framework structures wherein said process comprises providing preferably at a temperature between 50°C and 250°C and for a reaction time which is sufficient to obtain a crystalline product a reaction mixture composition expressed in terms of molar oxide ratios as follows:

$$sR:(M_xAl_yP_z)O_2:tH_2O$$

wherein "R" is an organic templating agent; "s" is the amount of "R" and has a value of from greater than zero to about 6; "t" has a value of from greater than zero to about 500; "M" represents at least two elements capable of forming a framework tetrahedral oxide where at least one element is selected from the group consisting of arsenic, beryllium, boron, chromium, gallium, germanium, lithium and vanadium and at least one element is selected from the group consisting of cobalt, iron, magnesium, manganese, titanium and zinc; and "x", "y" and "z" represent the mole fractions, respectively of "M", aluminum and phosphorus in the $(M_xAl_yP_z)O_2$ constituent, "y" and "z" each have a value of at least 0.01 and "x" has a value of at least 0.02, whereby the molecular sieves of claim 1 are prepared.

33. Process of claim 32 where "x", "y" and "z" are within the pentagonal compositional area defined by points F, G, H, I and J said points representing the following values for "x", "y" and "z":

| | Mole fraction | | |
|---|---|---|---|
| Point | x | y | z |
| F | 0.02 | 0.60 | 0.38 |
| G | 0.02 | 0.38 | 0.60 |
| H | 0.39 | 0.01 | 0.60 |
| I | 0.98 | 0.01 | 0.01 |
| J | 0.39 | 0.60 | 0.01 |

34. Process according to claim 32 wherein the source of phosphorus in the reaction mixture is orthophosphoric acid.

35. Process according to claim 32 wherein the source of phosphorus in the reaction mixture is orthophosphoric acid and the source of aluminum is at least one compound selected from the group of pseudo-boehmite and aluminum alkoxide.

36. Process according to claim 35 wherein the aluminum alkoxide is aluminum isopropoxide.

37. Process according to claim 32 wherein the source of "M" is selected from the group consisting of oxides, alkoxides, hydroxides, halides, nitrates, sulfates and carboxylates.

38. Process according to claim 33 wherein the organic templating agent is a quaternary ammonium or quaternary phosphonium compound having the formula

$$R'_4X^+$$

wherein X is nitrogen or phosphorus and each R' is an alkyl or aryl group containing from 1 to 8 carbon atoms.

39. Process according to claim 32 wherein the organic templating agent is an amine.

40. Process according to claim 32 wherein the templating agent is selected from the group consisting of tetrapropylammonium ion; tetraethylammonium ion; tripropylamine; triethylamine; triethanolamine; piperidine; cyclohexylamine; 2-methyl pyridine; N,N - dimethylbenzylamine; N,N - dimethylethanolamine; choline; N,N' - dimethylpiperazine; 1,4 - diazabicyclo - (2,2,2) octane; N - methyldiethanolamine; N - methylethanolamine; N - methylpiperidine; 3 - methylpiperidine; N - methylcyclohexylamine; 3 - methylpyridine; 4 - methylpyridine; quinuclidine; N,N' - dimethyl - 1,4 - diazabicyclo (2,2,2) octane ion; tetramethylammonium ion; tetrabutylammonium ion; tetrapentylammonium ion; di - n - butylamine; neopentylamine; di - n - pentylamine; isopropylamine; t - butylamine; ethylenediamine; pyrrolidine; 2 - imidazolidone; di - n - propylamine; and a polymeric quaternary ammonium salt $[(C_{14}H_{32}N_2)(OH)_2]_q$ wherein q is a value of at least 2.

41. Molecular sieve prepared by calcining the compositions of claim 1 or claim 2 at a temperature sufficiently high to remove at least a portion of the organic templating agent present in the intracrystalline pore system.

42. Process for separating molecular species from admixture with molecular species having a lesser degree of polarity which comprises contacting said mixture of molecular species with a molecular sieve of

claim 1 or claim 2 having pore diameter large enough to adsorb at least one of the more polar molecular species, said molecular sieve being at least partially activated whereby molecules of the more polar molecular species are selectively adsorbed into the intracrystalline pore system thereof.

43. Process for separating a mixture of molecular species having different kinetic diameters which comprises contacting said mixture with a molecular sieve of claim 1 or claim 2 having pore diameters large enough to adsorb at least one but not all molecular species of said mixture, said molecular sieve being at least partially activated whereby at least some molecules whose kinetic diameters are sufficiently small can enter the intracrystalline pore system thereof.

44. Process according to claim 42 wherein the more polar molecular species is water.

45. Process for converting a hydrocarbon which comprises contacting said hydrocarbon under hydrocarbon converting conditions with a molecular sieve of claim 1 or claim 2.

46. Process according to claim 45 wherein the hydrocarbon conversion process is cracking.

47. Process according to claim 45 wherein the hydrocarbon conversion process is hydrocracking.

48. Process according to claim 45 wherein the hydrocarbon conversion process is hydrogenation.

49. Process according to claim 45 wherein the hydrocarbon conversion process is polymerization.

50. Process according to claim 45 wherein the hydrocarbon conversion process is alkylation.

51. Process according to claim 45 wherein the hydrocarbon conversion process is reforming.

52. Process according to claim 45 wherein the hydrocarbon conversion process is isomerization.

53. Process according to claim 52 wherein the isomerization conversion process is xylene isomerization.

54. Process according to claim 45 wherein the hydrocarbon conversion process is dehydrocyclization.

**Patentansprüche**

1. Kristalline Molekularsiebe mit dreidimensionalen mikroporösen Gerüststrukturen aus tetraedrischen Einheiten aus $AlO_2^-$, $PO_2^+$ und $MO_2^n$ mit der Ladung n, worin "n" −3, −2, −1, 0 oder +1 sein kann mit einer empirischen chemischen Zusammensetzung auf wasserfreier Basis ausgedrückt durch die Formel:

$$mR:(M_xAl_yP_z)O_2$$

in der "R" wenigstens ein organisches Templatagens darstellt, das in dem intrakristallinen Porensystem vorhanden ist, "m" die molare Menge an "R" darstellt, die pro Mol $(M_xAl_yP_z)O_2$ vorhanden ist und einen Wert von zwischen 0 und etwa 0,3 hat, "M" wenigstens zwei Elemente darstellt, die in der Lage sind, tetraedrische Gerüstoxideinheiten zu bilden, umfassend wenigstens ein Element ausgewählt aus der Gruppe bestehend aus Arsen, Beryllium, Bor, Chrom, Gallium, Germanium, Lithium und Vanadium und wenigstens ein Element ausgewählt aus der Gruppe bestehend aus Kobalt, Magnesium, Mangan, Eisen, Titan und Zink, wobei "x", "y" und "z" die jeweiligen Molfraktionen von "M", Aluminium und Phosphor darstellen und innerhalb eines pentagonalen Zusammensetzungsgebiets liegen, das durch die Punkte A, B, C, D und E definiert wird, wobei diese Punkte die nachfolgenden Werte für "x", "y" und "z" darstellen:

| | Molfraktion | | |
|---|---|---|---|
| Punkte | x | y | z |
| A | 0.02 | 0.60 | 0.38 |
| B | 0.02 | 0.38 | 0.60 |
| C | 0.39 | 0.01 | 0.60 |
| D | 0.98 | 0.01 | 0.01 |
| E | 0.39 | 0.60 | 0.01 |

2. Kristalline Molekularsiebe nach Anspruch 1 bei denen "x", "y" und "z" innerhalb eines hexagonalen Zusammensetzungsgebiets liegen, das durch die Punkte a, b, c, d, e und f definiert wird, wobei diese Punkte die nachfolgenden Werte für "x", "y" und "z" darstellen:

| | Molfraktion | | |
|---|---|---|---|
| Punkte | x | y | z |
| a | 0.02 | 0.60 | 0.38 |
| b | 0.02 | 0.38 | 0.60 |
| c | 0.39 | 0.01 | 0.60 |
| d | 0.60 | 0.01 | 0.39 |
| e | 0.60 | 0.39 | 0.01 |
| f | 0.39 | 0.60 | 0.01 |

3. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 7.3—7.65 | 12.1—11.56 | m—sst |
| 19.5—19.95 | 4.55—4.46 | m—st |
| 20.9—21.3 | 4.25—4.17 | m—sst |
| 22.2—22.6 | 4.00—3.93 | schw—sst |
| 25.7—26.15 | 3.47—3.40 | schw—m |

4. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 9.3—9.65 | 9.51—9.17 | m—st |
| 20.2—20.6 | 4.40—4.31 | m—st |
| 20.9—21.3 | 4.25—4.17 | st—sst |
| 22.0—22.5 | 4.04—3.95 | m—st |
| 22.5—22.9 | 3.95—3.92 | m—st |
| 23.0—23.4 | 3.87—3.80 | m—sst |

5. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 8.6—8.9 | 10.3—9.93 | sst |
| 13.0 | 6.81 | schw |
| 21.9—22.2 | 4.06—4.00 | schw |
| 25.4 | 3.51 | schw |
| 27.5 | 3.24 | schw |
| 29.7 | 3.01 | schw |

6. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 11.3—11.6 | 7.83—7.63 | m—sst |
| 18.7—18.9 | 4.75—4.70 | schw—st |
| 21.9—22.3 | 4.06—3.99 | m—sst |
| 26.5—27.0 | 3.363—3.302 | schw—m |
| 29.7—30.05 | 3.008—2.974 | schw—m |

7. Kristalline Molekularsiebe nach den Ansprüchen 1 oder 2 mit einem charakteristischen Röntgenpulverbeugungsmuster das wenigstens die in der nachfolgenden Tabelle wiedergegebenen d-Werte enthält:

| 2θ | d(Å) | relative Intensität |
|---|---|---|
| 7.7—7.75 | 11.5—11.4 | sst |
| 13.4 | 6.61 | st—sst |
| 15.5—15.55 | 5.72—5.70 | st |
| 19.65—19.7 | 4.52—4.51 | schw—st |
| 20.5—20.6 | 4.33—4.31 | sst |
| 31.3—32.00 | 2.812—2.797 | schw—st |

8. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 9.6—9.65 | 9.21—9.16 | sst |
| 15.5—15.55 | 5.72—5.70 | m |
| 16.9—17.1 | 5.25—5.19 | m |
| 20.15—20.25 | 4.41—4.39 | m |
| 20.95—21.05 | 4.24—4.22 | m |
| 31.8—32.5 | 2.814—2.755 | m |

9. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 13.7—14.25 | 6.46—6.22 | m—sst |
| 19.55—20.0 | 4.54—4.44 | schw—st |
| 24.05—24.5 | 3.70—3.63 | m—sst |
| 34.3—35.0 | 2.614—2.564 | sschw—schw |
| 42.5—43.0 | 2.127—2.103 | sschw—schw |

10. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 8.5—8.6 | 10.40—10.28 | m—st |
| 20.2—20.3 | 4.40—4.37 | m |
| 21.9—22.1 | 4.06—4.02 | schw—m |
| 22.6—22.7 | 3.93—3.92 | sst |
| 31.7—31.8 | 2.823—2.814 | schw—m |

11. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 9.25—9.55 | 9.56—9.26 | schw—m |
| 12.5—12.9 | 7.08—6.86 | sst |
| 16.9—17.3 | 5.25—5.13 | schw—m |
| 20.45—20.9 | 4.34—4.25 | schw—m |
| 23.85—24.25 | 3.73—3.67 | schw—m |
| 26.05—26.35 | 3.42—3.38 | schw—m |
| 27.3—27.6 | 3.27—3.23 | sst |

12. Kristalline Molekularsiebe nach den Ansprüchen 1 oder 2 mit einem charakteristischen Röntgenpulverbeugungsmuster, das wenigstens die in der nachfolgenden Tabelle widergegebenen d-Werte enthält:

| 2θ | d(Å) | relative Intensität |
|---|---|---|
| 13.15—13.4 | 6.73—6.61 | sst |
| 18.05—18.35 | 4.91—4.83 | m |
| 18.4—18.6 | 4.82—4.77 | m |
| 26.55—26.7 | 3.36—3.34 | m |
| 32.0—32.1 | 2.80—2.79 | m |

13. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 9.4—9.65 | 9.41—9.17 | st—sst |
| 15.9—16.2 | 5.57—5.47 | sschw—m |
| 17.85—18.4 | 4.97—4.82 | schw—st |
| 20.3—20.9 | 4.37—4.25 | m—sst |
| 24.95—25.4 | 3.57—3.51 | sschw—st |
| 30.3—30.8 | 2.95—2.90 | schw—st |

14. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 10.8—11.1 | 8.19—7.97 | m |
| 17.2—17.4 | 5.16—5.10 | st—sst |
| 21.0—21.25 | 4.23—4.18 | m—st |
| 21.8—22.0 | 4.08—4.04 | sst |
| 31.8—32.2 | 2.814—2.788 | m |

15. Kristalline Molekularsiebe nach den Ansprüchen 1 oder 2 mit einem charakteristischen Röntgenpuvlerbeugungsmuster, das wenigstens die in der nachfolgenden Tabelle wiedergegebenen d-Werte enthält:

| 2θ | d(Å) | relative Intensität |
|---|---|---|
| 7.7—7.9 | 11.5—11.2 | sst |
| 16.2—16.6 | 5.47—5.34 | schw—m |
| 18.9—19.3 | 4.70—4.60 | m—st |
| 20.6—20.8 | 4.31—4.27 | schw—st |
| 21.8—22.0 | 4.08—4.04 | m |
| 22.2—22.5 | 4.00—3.95 | schw—m |

16. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 6.1—6.3 | 14.49—14.03 | sst |
| 15.5—15.7 | 5.72—5.64 | schw—m |
| 18.5—18.8 | 4.80—4.72 | schw—m |
| 23.5—23.7 | 3.79—3.75 | schw—m |
| 26.9—27.1 | 3.31—3.29 | schw—m |

17. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 9.4—9.6 | 9.41—9.21 | schw—m |
| 13.3—13.6 | 6.66—6.51 | m—sst |
| 18.0—18.4 | 4.93—4.82 | m |
| 21.2—21.5 | 4.19—4.13 | m—st |
| 22.5—23.0 | 3.95—3.87 | st—sst |
| 30.2—30.5 | 2.96—2.93 | schw—m |

18. Kristalline Molekularsiebe nach den Ansprüchen 1 oder 2 mit einem charakteristischen Röntgenpulverbeugungsmuster, das wenigstens die in der nachfolgenden Tabelle widergegebenen d-Werte enthält:

| 2θ | d(Å) | relative Intensität |
|---|---|---|
| 7.5—7.7 | 11.79—11.48 | sschw—m |
| 8.0—8.1 | 11.05—10.94 | st—sst |
| 12.4—12.5 | 7.14—7.08 | schw—sst |
| 13.6—13.8 | 6.51—6.42 | m—st |
| 14.0—14.1 | 6.33—6.28 | schw—m |
| 27.8—28.0 | 3.209—3.187 | schw—m |

19. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 13.6—13.8 | 6.51—6.42 | schw—m |
| 20.5—20.6 | 4.33—4.31 | schw—m |
| 21.1—21.3 | 4.21—4.17 | sst |
| 22.1—22.3 | 4.02—3.99 | m—st |
| 22.8—23.0 | 3.90—3.86 | m |
| 23.1—23.4 | 3.82—3.80 | schw—m |
| 25.5—25.9 | 3.493—3.440 | schw—m |

20. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 7.15—7.4 | 12.36—11.95 | m—sst |
| 12.5—12.7 | 7.08—6.97 | m—st |
| 21.75—21.9 | 4.09—4.06 | m—st |
| 24.1—24.25 | 3.69—3.67 | sst |
| 27.25—27.4 | 3.273—3.255 | st |
| 30.05—30.25 | 2.974—2.955 | m—st |

21. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 9.4—9.55 | 9.41—9.26 | sst |
| 13.0—13.1 | 6.81—6.76 | schw—m |
| 16.0—16.2 | 5.54—5.47 | schw—m |
| 20.6—20.85 | 4.31—4.26 | st—sst |
| 24.3—24.4 | 3.66—3.65 | schw—sst |
| 30.7—30.95 | 2.912—2.889 | schw—st |

22. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 7.2—8.1 | 12.3—10.9 | sst |
| 21.2—21.8 | 4.19—4.08 | schw—m |
| 22.5—23.0 | 3.95—3.87 | sschw—m |
| 26.6—27.2 | 3.351—3.278 | sschw—schw |
| 28.5—29.0 | 3.132—3.079 | sschw—schw |

23. Kristalline Molekularsiebe nach den Ansprüchen 1 oder 2 mit einem charakteristischen Röntgenpulverbeugungsmuster, das wenigstens die in der nachfolgenden Tabelle wiedergegebenen d-Werte enthält:

| 2θ | d(Å) | relative Intensität |
|---|---|---|
| 9.4 | 9.41 | sst |
| 15.9—16.0 | 5.57—5.54 | schw—m |
| 20.5—20.6 | 4.33—4.31 | st |
| 24.5—24.7 | 3.63—3.60 | schw |
| 25.8—25.9 | 3.45—3.44 | schw |
| 30.4—30.5 | 2.940—2.931 | schw |

24. Molekularsieb nach den Ansprüchen 1 oder 2 umfassend eine dreidimensionale mikroporöse Gerüststruktur aus tetraedrischen Einheiten von $MO_2^n$, $AlO_2^-$ und $PO_2^+$, bei dem "n" −2, −1, 0 oder +1 ist.

25. Molekularsiebe nach Anspruch 1, bei denen "M" Beryllium oder Magnesium ist.

26. Molekularsiebe nach Anspruch 1, bei denen "M" wenigstens eines aus der Gruppe bestehend aus Arsen, Germanium, Chrom und Vanadium ist und wenigstens eines aus der Gruppe bestehend aus Mangan, Magnesium, Eisen und Zink.

27. Molekularsiebe nach Anspruch 1, bei denen "M" wenigstens eines aus Bor und Gallium ist und wenigstens eines aus der Gruppe bestehend aus Eisen, Magnesium, Mangan und Zink.

28. Molekularsiebe nach Anspruch 1, bei denen "M" Magnesium und Germanium ist.

29. Molekularsiebe nach Anspruch 1, bei denen "M" wenigstens ein Element ausgewählt aus der Gruppe bestehend aus Beryllium und Germanium und wenigstens ein Element ausgewählt aus der Gruppe bestehend aus Magnesium, Mangan, Eisen und Zink ist.

30. Molekularsiebe nach Anspruch 26, bei denen "M" Chrom und Magnesium ist.

31. Molekularsiebe nach Anspruch 26, bei denen "M" Eisen und Arsen ist.

32. Verfahren zur Herstellung von kristallinen Molekularsieben nach Anspruch 1, die dreidimensionale mikroporöse Gerüststrukturen haben, wobei dieses Verfahren das Bereitstellen vorzugsweise bei einer Temperatur zwischen 50°C und 250°C und für eine Reaktionszeit die ausreichend ist ein kristallines Produkt zu erhalten, einer Reaktionsmischung mit einer Zusammensetzung zahlenmäßig ausgedrückt durch die Molverhältnisse der Oxide wie folgt umfaßt:

$$sR:(M_xAl_yP_z)O_2:tH_2O$$

in der "R" ein organisches Templatagens ist, "s" die Menge an "R" darstellt und einen Wert von größer als 0 bis etwa 6 hat, "t" einen Wert von größer als 0 bis etwa 500 hat und "M" jeweils zwei Elemente darstellt, die in der Lage sind, tetraedrische Gerüstoxide zu bilden, wobei wenigstens ein Element ausgewählt wird aus der Gruppe bestehend aus Arsen, Beryllium, Bor, Chrom, Gallium, Germanium, Lithium und Vanadium und wenigstens ein Element ausgewählt wird aus der Gruppe bestehend aus Kobalt, Eisen, Magnesium, Mangan, Titan und Zink und "x", "y" und "z" die jeweiligen Molfraktionen von "M", Aluminium und Phosphor in dem $(M_xAl_yP_z)O_2$ Bestandteil darstellen, wobei "y" und "z" jeweils einen Wert von wenigstens 0,01 haben und "x" einen Wert von wenigstens 0,02 hat und wobei die Molekularsiebe gemäß Anspruch 1 hergestellt werden.

33. Verfahren nach Anspruch 32, bei dem "x", "y" und "z" innerhalb eines pentagonalen Zusammensetzungsgebiets liegen, das durch die Punkte F, G, H, I und J definiert ist, wobei diese Punkte jeweils die nachfolgenden Werte für "x", "y" und "z" darstellen:

| | Molfraktion | | |
|---|---|---|---|
| Punkt | x | y | z |
| F | 0.02 | 0.60 | 0.38 |
| G | 0.02 | 0.38 | 0.60 |
| H | 0.39 | 0.01 | 0.60 |
| I | 0.98 | 0.01 | 0.01 |
| J | 0.39 | 0.60 | 0.01 |

34. Verfahren nach Anspruch 32, bei dem die Quelle für Phosphor in der Reaktionsmischung Orthophosphorsäure ist.

35. Verfahren nach Anspruch 32, bei dem die Quelle für Phosphor in der Reaktionsmischung Orthophosphorsäure ist und die Quelle für Aluminium wenigstens eine Verbindung ausgewählt aus der Gruppe Pseudoboehmit und Aluminiumalkoxid ist.

36. Verfahren nach Anspruch 35, bei dem das Aluminiumalkoxid Aluminiumisopropoxid ist.

37. Verfahren nach Anspruch 32, bei dem die Quelle für "M" ausgewählt wird aus der Gruppe bestehend aus den Oxiden, Alkoxiden, Hydroxiden, Halogeniden, Nitraten, Sulfaten und Carboxylaten.

38. Verfahren nach Anspruch 33, bei dem das organische Templatagens eine quaternäre Ammonium- oder quaternäre Phosphoniumverbindung ist mit der Formel

$$R'_4X^+$$

in der X Stickstoff oder Phosphor ist und R′ jeweils eine Alkyl- oder Arylgruppe mit 1 bis 8 Kohlenstoffatomen darstellt.

39. Verfahren nach Anspruch 32, bei dem das organische Templatagens ein Amin ist.

40. Verfahren nach Anspruch 32, bei dem das Templatagens ausgewählt wird aus der Gruppe bestehend aus: Tetrapropylammoniumion, Tetraethylammoniumion, Tripropylamin, Triethylamin, Triethanolamin, Piperidin, Cyclohexylamin, 2 - Methylpyridin, N,N - dimethylbenzylamin, N,N - dimethylethanolamin, Cholin, N,N′ - dimethylpiperazin, 1,4 - diazabicyclo - (2,2,2) - octan, N - methyldiethanolamin, N - methylethanolamin, N - methylpiperidin, 3 - methylpiperidin, N - methylcyclohexylamin, 3 - methylpyridin, 4 - methylpyridin, Chinuclidin, N,N′ - dimethyl - 1,4 - diazabicyclo(2,2,2) - octanion, Tetramethylammoniumion, Tetrabutylammoniumion, Tetrapentylammoniumion, Di - n - butylamin, Neopentylamin, Di - n - pentylamin, Isopropylamin, t - Butylamin, Ethylendiamin, Pyrrolidin, 2 - Imidazolidon, Di - n - propylamin und einem polymeren quaternären Ammoniumsalz $[(C_{14}H_{32}N_2)(OH)_2]_q$ bei dem q einen Wert von wenigstens 2 einnimmt.

41. Molekularsieb hergestellt durch Kalzinieren der Zusammensetzungen gemäß Anspruch 1 oder Anspruch 2 bei einer Temperatur die ausreichend hoch ist, um wenigstens einen Teil des organischen Templatagens, das in dem intrakristallinen Porensystem vorhanden ist, zu entfernen.

42. Verfahren zur Abtrennung molekularer Spezies aus einer Mischung mit molekularen Spezies, die einen geringeren Polaritätsgrad aufweisen, umfassend das In-Kontakt-Bringen dieser Mischung molekularer Spezies mit einem Molekularsieb gemäß Anspruch 1 oder Anspruch 2, das Porendurchmesser hat, die groß genug sind um wenigstens einen Teil der polareren molekularen Spezies zu adsorbieren, wobei das Molekularsieb wenigstens teilweise aktiviert ist, wobei Moleküle der polaren molekularen Spezies selektiv in das intrakristalline Porensystem des Molekularsiebs adsorbiert werden.

43. Verfahren zur Trennung einer Mischung molekularer Spezies mit verschiedenen kinetischen Durchmessern umfassend das In-Kontakt-Bringen dieser Mischung mit einem Molekularsieb gemäß Anspruch 1 oder Anspruch 2, das Porendurchmesser hat, die groß genug sind wenigstens eines jedoch nicht alle molekularen Spezies dieser Mischung zu adsorbieren, wobei dieses Molekularsieb wenigstens teilweise aktiviert wird, wobei wenigstens einige Moleküle deren kinetische Durchmesser ausreichend klein sind in das intrakristalline Porensystem des Molekularsiebs eindringen können.

44. Verfahren nach Anspruch 42, bei dem die polarere molekulare Spezies Wasser ist.

45. Verfahren zur Umwandlung eines Kohlenwasserstoffs umfassend das In-Kontakt-Bringen dieses Kohlenwasserstoffs unter Kohlenwasser-Umwandlungs-Bedingungen mit einem Molekularsieb gemäß Anspruch 1 oder Anspruch 2.

46. Verfahren nach Anspruch 45, bei dem der Kohlenwasserstoffumwandlungsprozess Cracken ist.

47. Verfahren nach Anspruch 45, bei dem der Kohlenwasserstoffumwandlungsprozess Hydrocracken ist.

48. Verfahren nach Anspruch 45, bei dem der Kohlenwasserstoffumwandlungsprozess eine Hydrogenierung ist.

49. Verfahren nach Anspruch 45, bei dem der Kohlenwasserstoffumwandlungsprozess eine Polymerisierung ist.

50. Verfahren nach Anspruch 45, bei dem der Kohlenwasserstoffumwandlungsprozess eine Alkylierung ist.

51. Verfahren nach Anspruch 45, bei dem der Kohlenwasserstoffumwandlungsprozess Reformieren ist.

52. Verfahren nach Anspruch 45, bei dem der Kohlenwasserstoffumwandlungsprozess eine Isomerisierung ist.

53. Verfahren nach Anspruch 52, bei dem der Isomerisierungs-Umwandlungsprozess eine Xylenisomerisierung ist.

54. Verfahren nach Anspruch 45, bei dem der Kohlenwasserstoffumwandlungsprozess eine Dehydrocyclisierung ist.

**Revendications**

1. Tamis moléculaire cristallin comportant une structure de base tridimensionnelle microporeuse formée par des motifs tétraédriques $AlO_2^-$, $PO_2^+$ et $MO_2^n$ avec une charge n, "n" pouvant être égal à −3, −2, −1, 0 ou +1, et ayant une composition chimique empirique représentée par la formule à l'état anhydre:

$$mR:(M_xAl_yP_z)O_2$$

dans laquelle "R" représente au moins un agent organique formant matrice, présent dans le système de pores intracristallins; "m" représente le nombre de moles de "R" présent par mole de $(M_xAl_yP_z)O_2$, et a une valeur variant de 0 à environ 0,3; "M" représente au moins deux éléments susceptibles de former des motifs oxyde téraédriques de base, et il comprend au moins un élément choisi parmi l'arsenic, le béryllium, le bore, le chrome, le gallium, le germanium, le lithium et le vanadium, et au moins un élément choisi parmi le cobalt, le magnésium, le manganèse, le fer, le titane et le zinc; "x", "y" et "z" représentent respectivement les fractions molaires de "M", d'aluminium et de phosphore, et elles sont comprises dans

un domaine pentagonal de composition, délimité par des points A, B, C, D et E, ces points correspondant aux valeurs de "x", "y" et "z" suivantes:

| | Fraction molaire | | |
|---|---|---|---|
| Point | x | y | z |
| A | 0,02 | 0,60 | 0,38 |
| B | 0,02 | 0,38 | 0,60 |
| C | 0,39 | 0,01 | 0,60 |
| D | 0,98 | 0,01 | 0,01 |
| E | 0,39 | 0,60 | 0,01 |

2. Tamis moléculaire cristallin selon la revendication 1, dans lequel "x", "y" et "z", sont situés dans un domaine hexagonal de composition, délimité par des points a, b, c, d, e et f, ces points correspondant aux valeurs de "x", "y" et "z" suivantes:

| | Fraction molaire | | |
|---|---|---|---|
| Point | x | y | z |
| a | 0,02 | 0,60 | 0,38 |
| b | 0,02 | 0,38 | 0,60 |
| c | 0,39 | 0,01 | 0,60 |
| d | 0,60 | 0,01 | 0,39 |
| e | 0,60 | 0,39 | 0,01 |
| f | 0,39 | 0,60 | 0,01 |

3. Tamis moléculaire cristallin selon la revendication 1 ou 2, ayant un diagramme caractéristique de diffraction de rayons X dans une poudre, faisant apparaître au moins les intervalles d mentionnés dans le tableau suivant:

| 2θ | d(Å) | Intensité relative* |
|---|---|---|
| 7,3—7,65 | 12,1—11,56 | m—vs |
| 19,5—19,95 | 4,55—4,46 | m—s |
| 20,9—21,3 | 4,25—4,17 | m—vs |
| 22,2—22,6 | 4,00—3,93 | w—vs |
| 25,7—26,15 | 3,47—3,40 | w—m |

* w: faible; m: moyenne; s: forte; vs: très forte.

4. Tamis moléculaire cristallin selon la revendication 1 ou 2, ayant un diagramme caractéristique de diffraction de rayons X dans une poudre, faisant apparaître au moins les intervalles d mentionnés dans le tableau suivant:

| 2θ | d(Å) | Intensité relative* |
|---|---|---|
| 9,3—9,65 | 9,51—9,17 | m—vs |
| 20,2—20,6 | 4,40—4,31 | m—s |
| 20,9—21,3 | 4,25—4,17 | s—vs |
| 22,0—22,5 | 4,04—3,95 | m—s |
| 22,5—22,9 | 3,95—3,92 | m—s |
| 23,0—23,4 | 3,87—3,80 | m—vs |

* m: moyenne; s: forte; vs: très forte.

5. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 8,6—8,9 | 10,3—9,93 | vs |
| 13,0 | 6,81 | w |
| 21,9—22,2 | 4,06—4,00 | w |
| 25,4 | 3,51 | w |
| 27,5 | 3,24 | w |
| 29,7 | 3,01 | w |

* w: faible; vs: très forte.

6. Tamis moléculaire cristallin selon la revendication 1 ou 2, ayant un diagramme caractéristique de diffraction de rayons X dans un poudre, qui fait apparaître au moins les intervalles d mentionnés dans le tableau suivant:

| 2θ | d(Å) | Intensité relative* |
|---|---|---|
| 11,3—11,6 | 7,83—7,63 | m—vs |
| 18,7—18,9 | 4,75—4,70 | w—s |
| 21,9—22,3 | 4,06—3,99 | m—vs |
| 26,5—27,0 | 3,363—3,302 | w—m |
| 29,7—30,05 | 3,008—2,974 | w—m |

* w: faible; m: moyenne; s: forte; vs: très forte.

7. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 7,7—7,75 | 11,5—11,4 | vs |
| 13,4 | 6,61 | s—vs |
| 15,5—15,55 | 5,72—5,70 | s |
| 19,65—19,7 | 4,52—4,51 | w—s |
| 20,5—20,6 | 4,33—4,31 | vs |
| 31,8—32,00 | 2,812—2,797 | w—s |

* w: faible; s: forte; vs: très forte.

8. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 9,6—9,65 | 9,21—9,16 | vs |
| 15,5—15,55 | 5,72—5,70 | m |
| 16,9—17,1 | 5,25—5,19 | m |
| 20,15—20,25 | 4,41—4,39 | m |
| 20,95—21,05 | 4,24—4,22 | m |
| 31,8—32,5 | 2,814—2,755 | m |

* w: faible; s: forte; vs: très forte.

9. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 13,7—14,25 | 6,46—6,22 | m—vs |
| 19,55—20,0 | 4,54—4,44 | w—s |
| 24,05—24,5 | 3,70—3,63 | m—vs |
| 34,3—35,0 | 2,614—2,564 | vw—w |
| 42,5—43,0 | 2,127—2,103 | vw—w |

* vw: très faible; w: faible; m: moyenne; s: forte; vs: très forte.

10. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 8,5—8,6 | 10,40—10,28 | m—s |
| 20,2—20,3 | 4,40—4,37 | m |
| 21,9—22,1 | 4,06—4,02 | w—m |
| 22,6—22,7 | 3,93—3,92 | vs |
| 31,7—31,8 | 2,823—2,814 | w—m |

* w: faible; m: moyenne; s: forte; vs: très forte.

11. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 9,25—9,55 | 9,56—9,26 | w—m |
| 12,5—12,9 | 7,08—6,86 | vs |
| 16,9—17,3 | 5,25—5,13 | w—m |
| 20,45—20,9 | 4,34—4,25 | w—m |
| 23,85—24,25 | 3,73—3,67 | w—m |
| 26,05—26,35 | 3,42—3,38 | w—m |
| 27,3—27,6 | 3,27—3,23 | vs |

* w: faible; m: moyenne; vs: très forte.

12. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 13,15—13,4 | 6,73—6,61 | vs |
| 18,05—18,35 | 4,91—4,83 | m |
| 18,4—18,6 | 4,82—4,77 | m |
| 26,55—26,7 | 3,36—3,34 | m |
| 32,0—32,1 | 2,80—2,79 | m |

* m: moyenne; vs: très forte.

13. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 9,4—9,65 | 9,41—9,17 | s—vs |
| 15,9—16,2 | 5,57—5,47 | vw—m |
| 17,85—18,4 | 4,97—4,82 | w—s |
| 20,3—20,9 | 4,37—4,25 | m—ws |
| 24,95—25,4 | 3,57—3,51 | vw—s |
| 30,3—30,8 | 2,95—2,90 | w—s |

* vw: très faible; w: faible; m: moyenne; s: forte; vs: très forte.

14. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 10,8—11,1 | 8,19—7,97 | m |
| 17,2—17,4 | 5,16—5,10 | s—vs |
| 21,0—21,25 | 4,23—4,18 | m—s |
| 21,8—22,0 | 4,08—4,04 | vs |
| 31,8—32,2 | 2,814—2,788 | m |

* m: moyenne; s: forte; vs: très forte.

15. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 7,7—7,9 | 11,5—11,2 | vs |
| 16,2—16,6 | 5,47—5,34 | w—m |
| 18,9—19,3 | 4,70—4,60 | m—v |
| 20,6—20,8 | 4,31—4,27 | w—s |
| 21,8—22,0 | 4,08—4,04 | m |
| 22,2—22,5 | 4,00—3,95 | w—m |

* w: faible; m: moyenne; s: forte; vs: très forte.

16. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 6,1—6,3 | 14,49—14,03 | vs |
| 15,5—15,7 | 5,72—5,64 | w—m |
| 18,5—18,8 | 4,80—4,72 | w—m |
| 23,5—23,7 | 3,79—3,75 | w—m |
| 26,9—27,1 | 3,31—3,29 | w—m |

* w: faible; m: moyenne; vs: très forte.

17. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 9,4—9,6 | 9,41—9,21 | w—m |
| 13,3—13,6 | 6,66—6,51 | m—vs |
| 18,0—18,4 | 4,93—4,82 | m |
| 21,2—21,5 | 4,19—4,13 | m—s |
| 22,5—23,0 | 3,95—3,87 | s—vs |
| 30,2—30,5 | 2,96—2,93 | w—m |

* w: faible; m: moyenne; s: forte; vs: très forte.

18. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 7,5—7,7 | 11,79—11,48 | vw—m |
| 8,0—8,1 | 11,05—10,94 | s—vs |
| 12,4—12,5 | 7,14—7,08 | w—vs |
| 13,6—13,8 | 6,51—6,42 | m—s |
| 14,0—14,1 | 6,33—6,28 | w—m |
| 27,8—28,0 | 3,209—3,187 | w—m |

* vw: très faible; w: faible; m: moyenne; s: forte; vs: très forte.

19. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 13,6—13,8 | 6,51—6,42 | w—m |
| 20,5—20,6 | 4,33—4,31 | w—m |
| 21,1—21,3 | 4,21—4,17 | vs |
| 22,1—22,3 | 4,02—3,99 | m—s |
| 22,8—23,0 | 3,90—3,86 | m |
| 23,1—23,4 | 3,82—3,80 | w—m |
| 25,5—25,9 | 3,493—3,440 | w—m |

* w: faible; m: moyenne; s: forte; vs: très forte.

20. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 7,15—7,4 | 12,36—11,95 | m—vs |
| 12,5—12,7 | 7,08—6,97 | m—s |
| 21,75—21,9 | 4,09—4,06 | m—s |
| 24,1—24,25 | 3,69—3,67 | vs |
| 27,25—27,4 | 3,273—3,255 | s |
| 30,05—30,25 | 2,974—2,955 | m—s |

* m: moyenne; s: forte; vs: très forte.

21. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
|---|---|---|
| 9,4—9,55 | 9,41—9,26 | vs |
| 13,0—13,1 | 6,81—6,76 | w—m |
| 16,0—16,2 | 5,54—5,47 | m—m |
| 20,6—20,85 | 4,31—4,26 | s—vs |
| 24,3—24,4 | 3,66—3,65 | w—vs |
| 30,7—30,95 | 2,912—2,889 | w—s |

* w: faible; m: moyenne; s: forte; vs: très forte.

22. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
| --- | --- | --- |
| 7,2—8,1 | 12,3—10,9 | vs |
| 21,2—21,8 | 4,19—4,08 | w—m |
| 22,5—23,0 | 3,95—3,87 | vw—m |
| 26,6—27,2 | 3,351—3,278 | vw—w |
| 28,5—29,0 | 3,132—3,079 | vw—w |

* vw: très faible; w: faible; m: moyenne; vs: très forte.

23. Tamis moléculaire cristallin selon la revendication 1 ou 2, ayant un diagramme caractéristique de diffraction de rayons X dans une poudre, qui fait apparaître au moins les intervalles d mentionnés dans le tableau suivant:

| 2θ | d(Å) | Intensité relative* |
| --- | --- | --- |
| 9,4 | 9,41 | vs |
| 15,9—16,0 | 5,57—5,54 | w—m |
| 20,5—20,6 | 4,33—4,31 | s |
| 24,5—24,7 | 3,63—3,60 | w |
| 25,8—25,9 | 3,45—3,44 | w |
| 30,4—30,5 | 2,940—2,931 | w |

* w: faible; m: moyenne; s: forte; vs: très forte.

24. Tamis moléculaire selon la revendication 1 ou 2, comprenant une structure de base tridimensionnelle microporeuse formée par des motifs tétraédriques $MO_2^n$, $AlO_2^-$ et $PO_2^+$, "n" étant égal à −2, −1, 0 ou +1.

25. Tamis moléculaire selon la revendication 1, dans lequel "M" représente le béryllium et le magnésium.

26. Tamis moléculaire selon la revendication 1, dans lequel "M" représente au moins un élément choisi parmi l'arsenic, le germanium, le chrome et le vanadium, et au moins un élément choisi parmi le manganèse, le magnésium, le fer et le zinc.

27. Tamis moléculaire selon la revendication 1, dans lequel "M" représente au moins un élément choisi parmi le bore et le gallium, et au moins un élément choisi parmi le fer, le magnésium, le manganèse et le zinc.

28. Tamis moléculaire selon la revendication 1, dans lequel "M" représente le magnésium et le germanium.

29. Tamis moléculaire selon la revendication 1, dans lequel "M" représente au moins un élément choisi parmi le béryllium et le germanium, et au moins un élément choisi parmi le magnésium, le manganèse, le fer et le zinc.

30. Tamis moléculaire selon la revendication 26, dans lequel "M" représente le chrome et le magnésium.

31. Tamis moléculaire selon la revendication 26, dans lequel "M" représente le fer et l'arsenic.

32. Procédé de préparation du tamis moléculaire cristallin de la revendication 1, ayant une structure tridimensionnelle microporeuse de base, dans lequel on fait réagir à une température de 50°C à 250°C pendant un temps suffisant pour obtenir un produit cristallin, un mélange réactionnel correspondant à la composition suivante exprimée en rapports molaires d'oxyde:

$$sR:(M_xAl_yP_z)O_2:tH_2O$$

dans laquelle "R" représente un agent organique formant matrice; "s" représente la quantité de "R", et a une valeur supérieure à zéro jusqu'à environ 6; "t" a une valeur supérieure à zéro jusqu'à environ 500; "M" représente au moins deux éléments susceptibles de former un oxyde téraédrique de base comprenant au moins un élément choisi parmi l'arsenic, le béryllium, le bore, le chrome, le gallium, le germanium, le lithium et le vanadium, et au moins un élément choisi parmi le cobalt, le fer, le magnésium, le manganèse, le titane et le zinc; et "x", "y" et "z" représentent respectivement les fractions molaires de "M", d'aluminium et de phosphore, dans le constituant $(M_xAl_yP_z)O_2$, "y" et "z" ayant chacun une valeur d'au moins 0,01, et "x" ayant une valeur d'au moins 0,02, afin d'obtenir le tamis moléculaire de la revendication 1.

33. Procédé selon la revendication 32, dans lequel "x", "y" et "z" sont compris dans le domaine pentagonal de composition, délimité par les points F, G, H, I et J, ces points représentant les valeurs de "x", "y" et "z" suivantes:

| | Fraction molaire | | |
|---|---|---|---|
| Point | x | y | z |
| F | 0,02 | 0,60 | 0,38 |
| G | 0,02 | 0,38 | 0,60 |
| H | 0,39 | 0,01 | 0,60 |
| I | 0,98 | 0,01 | 0,01 |
| J | 0,39 | 0,60 | 0,01 |

34. Procédé selon la revendication 32, dans lequel la source de phosphore dans le mélange réactionnel, est l'acide orthophosphorique.

35. Procédé selon la revendication 32, dans lequel la source de phosphore dans le mélange réactionnel, est l'acide orthophosphorique, et dans lequel la source d'aluminium, comprend au moins un composé choisi parmi la pseudo-boehmite et un alcoolate d'aluminium.

36. Procédé selon la revendication 35, dans lequel l'alcoolate d'aluminium, est l'isopropylate d'aluminium.

37. Procédé selon la revendication 32, dans lequel la source de "M", est choisie parmi les oxydes, les alcoolates, les hydroxydes, les halogénures, les nitrates, les sulfates et les carboxylates.

38. Procédé selon la revendication 33, dans lequel l'agent organique formant matrice, est un composé dérivé d'ammonium quaternaire ou de phosphonium quaternaire, de formule:

$$R_4'X^+$$

dans laquelle X représente un atome d'azote ou de phosphore, et chaque groupe R' représente un groupe alkyle ou aryle contenant de 1 à 8 atomes de carbone.

39. Procédé selon la revendication 32, dans lequel l'agent organique formant matrice, est une amine.

40. Procédé selon la revendication 32, dans lequel l'agent formant matrice est choisi parmi l'ion tétrapropylammonium, l'ion tétraéthylammonium, la tripropylamine, la triéthylamine, la triéthanolamine, la pipéridine, la cyclohexylamine, la 2 - méthyl pyridine, la N,N - diméthylbenzylamine, la N,N - diméthyléthanolamine, la choline, la N,N' - diméthylpipérazine, le 1,4 - diazabicyclo - (2,2,2) - octane, la N - méthyldiéthanolamine, la N - méthyléthanolamine, la N - méthylpipéridine, la 3 - méthylpipéridine, la N - méthylcyclohexylamine, la 3 - méthylpyridine, la 4 - méthylpyridine, la quinuclidine, l'ion N,N' - diméthyl - 1,4 - diazabicyclo (2,2,2) octane, l'ion tétraméthylammonium, l'ion tétrabutylammonium, l'ion tétrapentylammonium, la di - n - butylamine, la néopentylamine, la di - n - pentylamine, l'isopropylamine, la tert - butylamine, l'éthylènediamine, la pyrrolidine, la 2 - imidazolidone, la di - n - propylamine, et un sel d'ammonium quaternaire polymère de formule $[(C_{14}H_{32}N_2)(OH)_2]_q$ dans laquelle q est au moins égal à 2.

41. Tamis moléculaire préparé par calcination des compositions selon la revendication 1 ou la revendication 2, à une température suffisamment élevée pour éliminer au moins une partie de l'agent organique formant matrice présent dans le système de pores intracristallins.

42. Procédé de séparation d'espèces moléculaires à partir d'un mélange contenant des espèces moléculaires ayant un plus faible degré de polarité, dans lequel on met en contact ce mélange d'espèces moléculaires, avec un tamis moléculaire selon la revendication 1 ou la revendication 2, ayant un diamètre de pores suffisamment grand pour adsorber au moins une des espèces moléculaires les plus polaires, ce tamis moléculaire étant au moins partiellement activé, de sorte que les molécules des espèces moléculaires les plus polaires, sont sélectivement adsorbées dans son système de pores intracristallins.

43. Procédé de séparation d'un mélange d'espèces moléculaires ayant différents diamètres cinétiques, dans lequel on met en contact ce mélange avec un tamis moléculaire selon la revendication 1 ou la revendication 2, comportant des pores d'un diamètre suffisamment important pour adsorber au moins une, mais pas la totalité des espèces moléculaires de ce mélange, ce tamis moléculaire étant au moins partiellement activé, de sorte qu'au moins plusieurs molécules dont les diamètres cinétiques sont suffisamment petits, peuvent pénétrer dans son système de pores intracristallins.

44. Procédé selon la revendication 42, dans lequel l'espèce moléculaire la plus polaire est l'eau.

45. Procédé de conversion d'un hydrocarbure, dans lequel on met en contact cet hydrocarbure dans des conditions de conversion d'hydrocarbure, avec un tamis moléculaire selon la revendication 1 ou la revendication 2.

46. Procédé selon la revendication 45, dans lequel l'opération de conversion de l'hydrocarbure, est un craquage.

47. Procédé selon la revendication 45, dans lequel l'opération de conversion de l'hydrocarbure, est un hydrocraquage.

48. Procédé selon la revendication 45, dans lequel l'opération de conversion de l'hydrocarbure, est une hydrogénation.

49. Procédé selon la revendication 45, dans lequel l'opération de conversion de l'hydrocarbure, est une polymérisation.

50. Procédé selon la revendication 45, dans lequel l'opération de conversion de l'hydrocarbure, est une alkylation.

51. Procédé selon la revendication 45, dans lequel l'opération de conversion de l'hydrocarbure, est un reformage.

52. Procédé selon la revendication 45, dans lequel l'opération de conversion de l'hydrocarbure, est une isomérisation.

53. Procédé selon la revendication 52, dans lequel l'opération de conversion par isomérisation, est une isomérisation de xylène.

54. Procédé selon la revendication 45, dans lequel l'opération de conversion de l'hydrocarbure, est une déshydrocyclisation.

FIG. 1

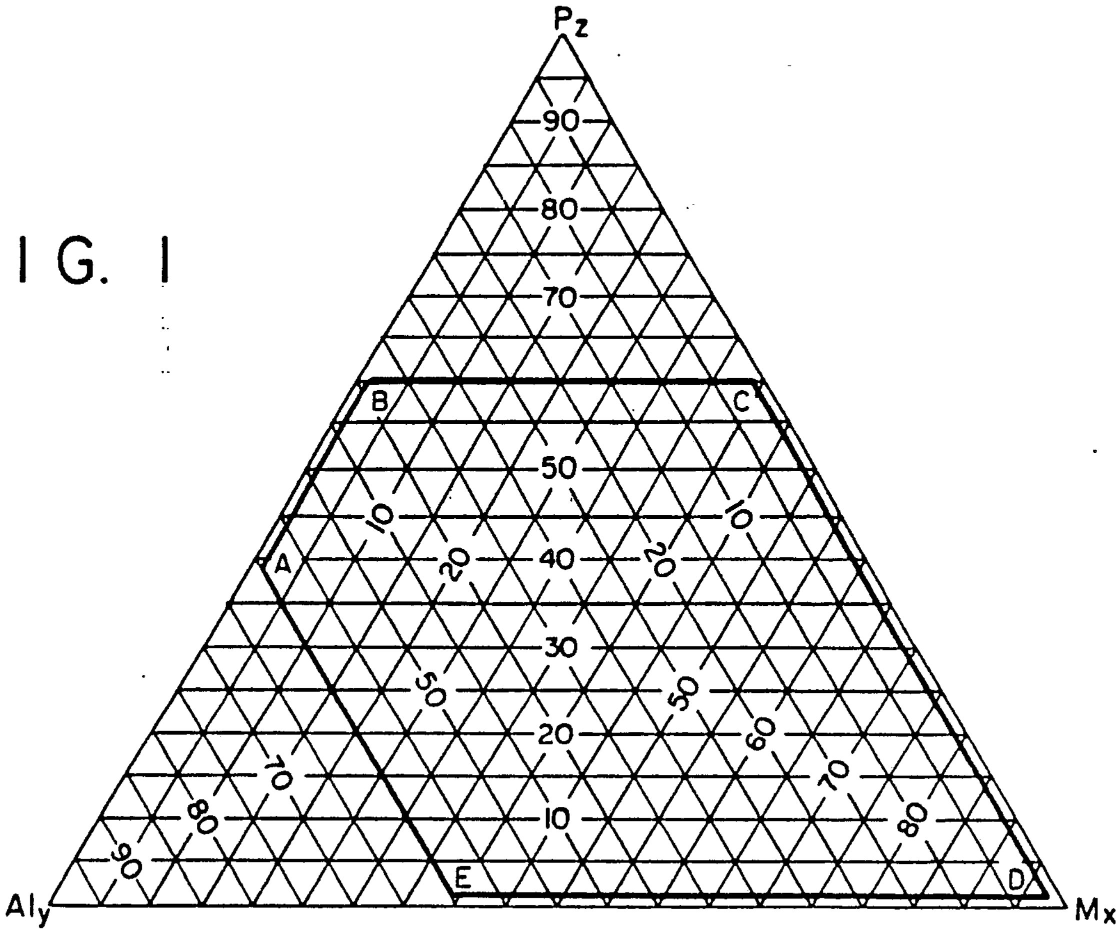
Pz
90
80
70
B
C
50
10
10
A
20
40
20
30
50
50
20
60
70
70
80
10
80
90
E
D
Aly
Mx

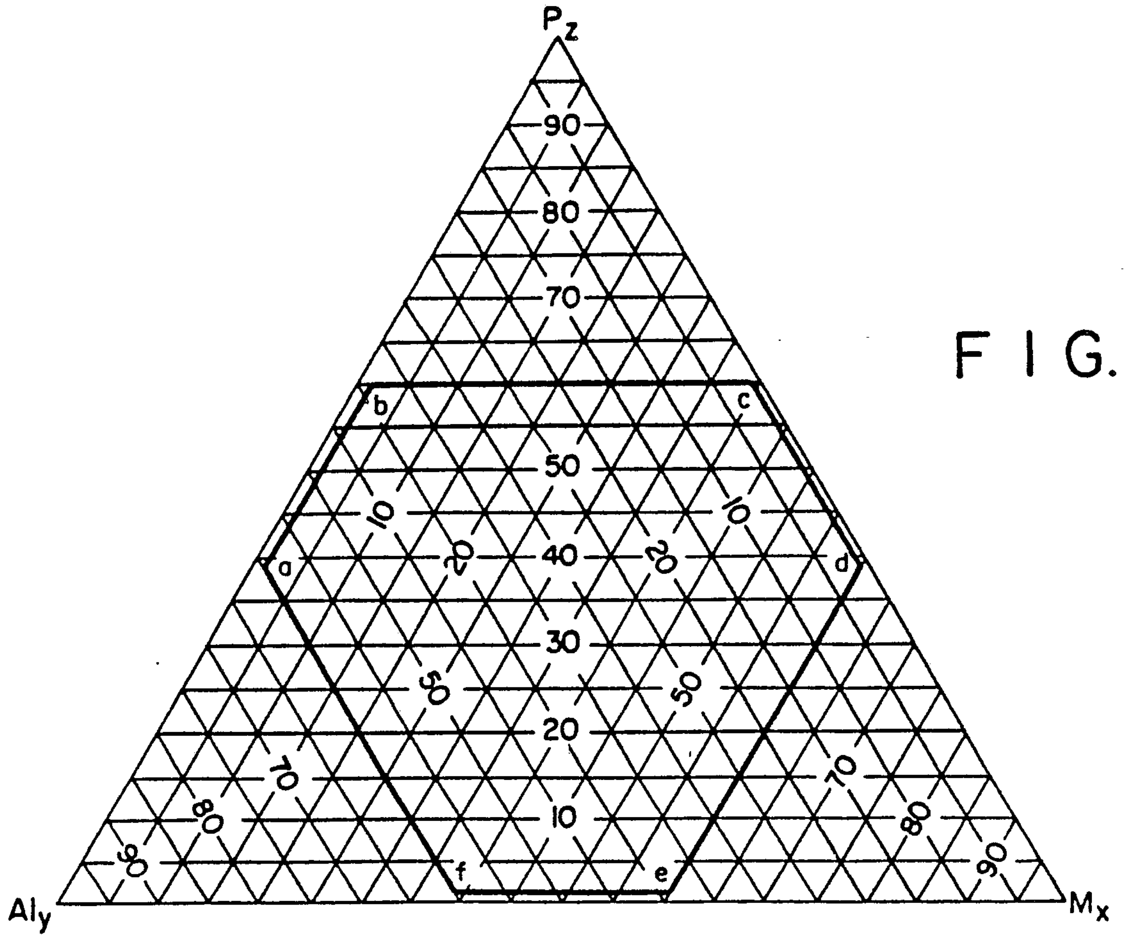
Pz
90
80
70
b
c
50
10
10
a
20
40
20
d
30
50
50
20
70
70
80
10
80
90
f
e
90
Aly
Mx

FIG. 2

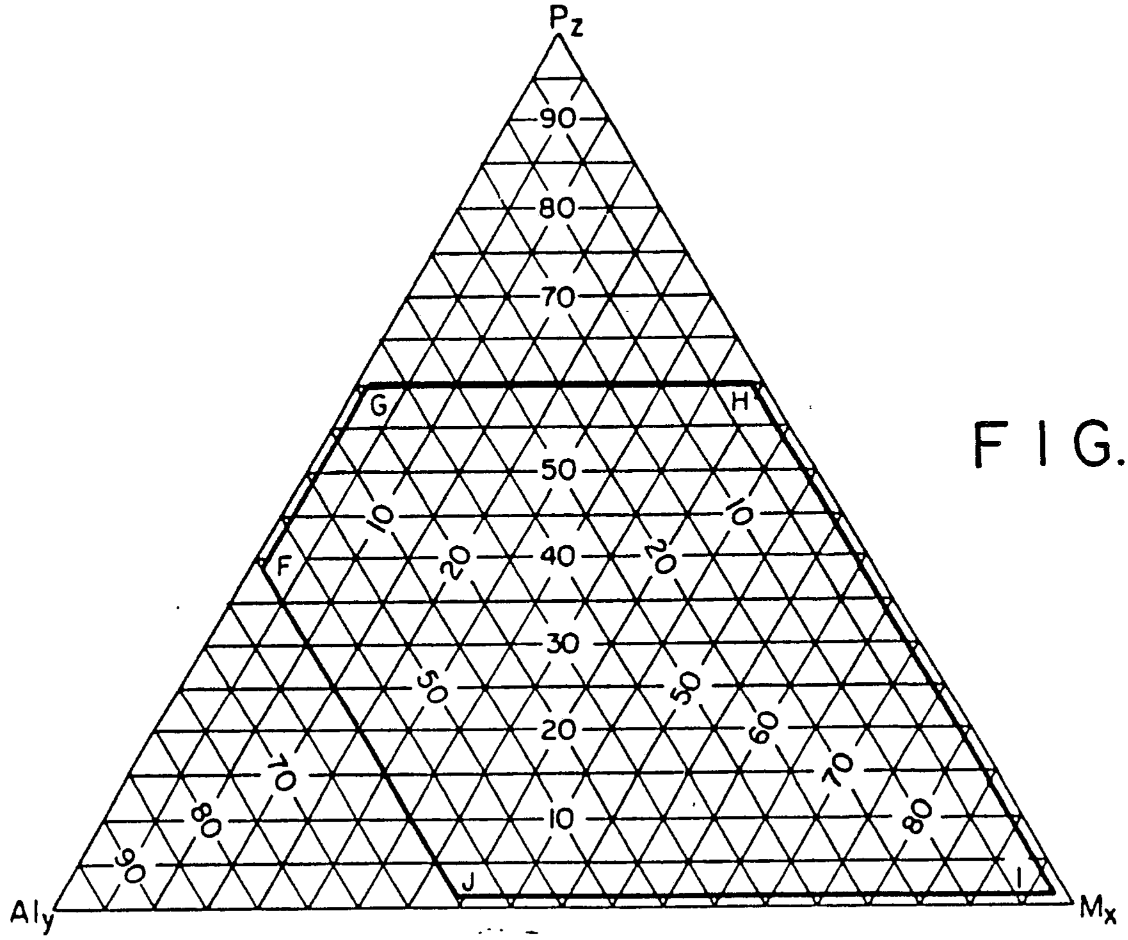
Pz
90
80
70
G
H
50
10
10
F
20
40
20
30
50
50
20
60
70
70
80
10
80
90
J
I
Aly
Mx

FIG. 3